# EUROPEAN PATENT APPLICATION

(11) **EP 4 311 488 A1**
(43) Date of publication of application: **31.01.2024**
(21) Application number: 23745369.1
(22) Date of filing: 24.03.2023
(51) Int. Cl.: A61B 5/11, A61B 5/00

(54) **ELECTRONIC DEVICE AND WEARABLE DEVICE FOR PROVIDING EVALUATION INFORMATION ON USER'S EXERCISE MOTION, AND METHOD FOR OPERATING SAME**

(30) Priority: 10.06.2022 KR 20220070958; 05.10.2022 KR 20220127283
(71) Applicant: Samsung Electronics Co., Ltd., Suwon-si, Gyeonggi-do 16677 (KR)
(72) Inventor: CHO, Heeyoung, Suwon-si, Gyeonggi-do 16677 (KR); KIM, Harkjoon, Suwon-si, Gyeonggi-do 16677 (KR); BAE, Soojung, Suwon-si, Gyeonggi-do 16677 (KR); LEE, Sangyoon, Suwon-si, Gyeonggi-do 16677 (KR); KIM, Sugyeong, Suwon-si, Gyeonggi-do 16677 (KR); AHN, Chiyoung, Suwon-si, Gyeonggi-do 16677 (KR); KIM, Jisu, Suwon-si, Gyeonggi-do 16677 (KR); KIM, Philgu, Suwon-si, Gyeonggi-do 16677 (KR)
(74) Representative: Gulde & Partner
(86) International application number: PCT/KR2023/003975
(87) International publication number: WO 2023/239025

(57) **Abstract**

An electronic device and a wearable device providing exercise posture evaluation information, and operation methods thereof are disclosed. The electronic device includes: an input module configured to receive a user input that selects an exercise program to be performed by a user wearing the wearable device; a communication module configured to receive, from the wearable device, sensor data including movement information of the user during an exercise performed by the user according to the selected exercise program; a processor configured to determine an exercise posture measurement value of the user based on the sensor data, and generate a first visual guide object including a first indicator indicating an exercise posture reference for the selected exercise program and at least one second indicator indicating the exercise posture measurement value; and a display module configured to output a graphical user interface (GUI) on which the first visual guide object is displayed.

## Description

### BACKGROUND

### 1. Field

Certain example embodiments relate to a wearable device and/or an electronic device for providing exercise posture evaluation information of a user, and/or operation methods of the electronic device and/or the wearable device.

### 2. Description of Related Art

A walking assistance device may generally be used, for example, to assist, with a walking exercise for rehabilitation or other purposes, a user who has a hard time walking by themselves due to some reasons, for example, diseases or accidents. Recent aging societies have contributed to a growing number of people who experience inconvenience and pain when walking from reduced muscular strength or joint problems due to aging, and there is thus a growing interest in walking assistance devices. A walking assistance device may be worn on a body of a user to provide the user with the power needed for the user to walk, to assist the user with walking in a normal walking pattern, and/or to aid in exercise.

### SUMMARY

According to an example embodiment, an electronic device may include: an input module, comprising input circuitry, configured to receive a user input that selects an exercise program to be performed by a user wearing a wearable device; a communication module, comprising communication circuitry, configured to receive, from the wearable device, sensor data including movement information of the user during an exercise performed by the user according to the selected exercise program; at least one processor configured to determine an exercise posture measurement value of the user based on the sensor data, and generate a first visual guide object including a first indicator indicating an exercise posture reference for the selected exercise program and at least one second indicator indicating the determined exercise posture measurement value; and a display module, comprising a display, configured to output a graphical user interface (GUI) on which the first visual guide object is displayed.

According to an example embodiment, an operation method of an electronic device may include: receiving a user input that selects an exercise program to be performed by a user wearing a wearable device; receiving, from the wearable device, sensor data including movement information of the user during an exercise performed by the user according to the selected exercise program; determining an exercise posture measurement value of the user based on the sensor data; and outputting a GUI on which a first visual guide object including a first indicator indicating an exercise posture reference for the selected exercise program and at least one second indicator indicating the determined exercise posture measurement value is displayed.

According to an example embodiment, a non-transitory computer-readable storage medium storing instructions that, when executed by a processor, cause the processor to perform the operation method of the electronic device to described herein.

Other features and aspects will be apparent from the following detailed description, the drawings, and the claims.

### BRIEF DESCRIPTION OF THE DRAWINGS

The above and other aspects, features, and advantages of certain embodiments of the present disclosure will be more apparent from the following detailed description, taken in conjunction with the accompanying drawings, in which:
FIG. 1 is a diagram illustrating an overview of a wearable device worn on a body of a user according to an example embodiment;
FIG. 2 is a diagram illustrating an example of an exercise management system including a wearable device and an electronic device according to an example embodiment;
FIG. 3 is a rear view of an example of a wearable device according to an example embodiment;
FIG. 4 is a left side view of an example of a wearable device according to an example embodiment;
FIGS. 5A and 5B are diagrams illustrating example configurations of a control system of a wearable device according to an example embodiment;
FIG. 6 is a diagram illustrating an example of an interaction between a wearable device and an electronic device according to an example embodiment;
FIG. 7 is a diagram illustrating an example configuration of an electronic device according to an example embodiment;
FIG. 8 is a flowchart illustrating an example of an operation method of an electronic device and a wearable device according to an example embodiment;
FIG. 9 is a flowchart illustrating an example of a method of providing exercise posture evaluation information of a user according to an example embodiment;
FIG. 10 is a diagram illustrating an example of providing exercise posture evaluation information of a user through a graphical user interface (GUI) of an electronic device and through a wearable device according to an example embodiment;
FIG. 11 is a diagram illustrating an example of evaluating an exercise posture of a user according to an example embodiment;
FIG. 12 is a diagram illustrating an example of a screen of a GUI including visual guide objects according to an example embodiment;
FIGS. 13A, 13B, and 13C are diagrams illustrating various examples of a first visual guide object according to an example embodiment;
FIGS. 14A and 14B are diagrams illustrating examples of a second visual guide object according to an example embodiment;
FIG. 15 is a diagram illustrating various examples of a second visual guide object based on a physical movement stability evaluation for a user according to an example embodiment;
FIG. 16 is a diagram illustrating an example of a change in visual guide objects by an exercise performed by a user according to an example embodiment;
FIG. 17 is a diagram illustrating an example of a first visual guide object providing exercise posture evaluation information of a user in the form of a gauge according to an example embodiment;
FIG. 18 is a diagram illustrating an example of a change in an avatar object in a first visual guide obj ect based on an exercise posture of a user according to an example embodiment;
FIG. 19 is a diagram illustrating an example of providing exercise posture evaluation information of a user through an avatar object and a wearable device according to an example embodiment; and
FIGS. 20A and 20B are diagrams illustrating examples of providing a GUI including visual guide objects through various electronic devices according to an example embodiment.

### DETAILED DESCRIPTION

The following detailed structural or functional description is provided merely as an example and various alterations and modifications may be made to the examples. Accordingly, actual implementations are not construed as limited to certain example embodiments of the disclosure and should be understood to include all changes, equivalents, and replacements within the idea and the technical scope of the disclosure.

As used herein, the singular forms "a," "an," and "the" include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or populations thereof.

Unless otherwise defined, all terms, including technical and scientific terms, used herein have the same meaning as commonly understood by those having ordinary skill in the art to which this disclosure pertains. Terms, such as those defined in commonly used dictionaries, are to be interpreted as having a meaning that is consistent with their meaning in the context of the relevant art, and are not to be interpreted in an idealized or overly formal sense unless expressly so defined herein.

Hereinafter, example embodiments will be described in detail with reference to the accompanying drawings. When describing the example embodiments with reference to the accompanying drawings, like reference numerals refer to like elements and a repeated description related thereto will be omitted.

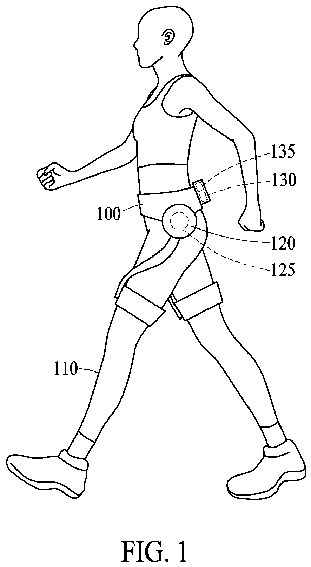

FIG. 1 is a diagram illustrating an overview of a wearable device worn on a body of a user according to an example embodiment.

Referring to FIG. 1, a wearable device 100 may be a device that is worn on a body of a user 110 to assist the user 110 in walking (and/or gait), doing an exercise, and/or working more readily. In an example embodiment, the wearable device 100 may be used to measure a physical ability (e.g., a walking ability, an exercise ability, and/or an exercise posture) of the user 110. The term "wearable device" used herein may be replaced with a "wearable robot," "walking assistance device," or "an exercise assistance device." The user 110 may be a human or an animal, but examples of which are not limited thereto. The wearable device 100 may be worn on the body (e.g., a lower body (e.g., legs, ankles, knees, etc.), an upper body (a torso, arms, wrists, etc.), or a waist) of the user 110 to apply an assistance force and/or a resistance force to a physical movement of the user 110. The assistance force, which is a force applied in the same direction as that of a physical movement of the user 110, may assist the user 110 in performing the physical movement. The resistance force, which is a force applied in a direction opposite to that of a physical movement of the user 110, may hinder the user 110 from performing the physical movement and may also be referred to as an "exercise load."

In an example embodiment, the wearable device 100 may operate in a walking assistance mode to assist the user 110 in walking. In the walking assistance mode, the wearable device 100 may assist the user 110 in walking by applying, to the body of the user 110, an assistance force generated from a driving module 120 of the wearable device 100. As the wearable device 100 assists the user 110 with a force required for the user 110 to walk, it may enable the user 110 to walk independently or walk for a longer period of time and may thereby increase a walking ability of the user 110. The wearable device 100 may also improve a gait of a user with an abnormal walking habit or walking posture.

In an example embodiment, the wearable device 100 may operate in an exercise assistance mode to enhance an exercise effect of the user 110. In the exercise assistance mode, the wearable device 100 may hinder the user 110 from performing a physical movement or apply resistance to a physical movement of the user 110 by applying, to the body of the user 110, a resistance force generated from the driving module 120 which comprises driving circuitry. For example, when the wearable device 100 is a hip-type wearable device that is to be worn on the waist (or pelvis) and legs (e.g., thighs) of the user 110, the wearable device 100 may provide an exercise load to a leg movement of the user 110 while being worn on legs of the user 110 and may thereby enhance further an exercise effect on the legs of the user 110. In an example embodiment, the wearable device 100 may apply the assistance force to the body of the user 110 to assist the user 110 in doing an exercise. For example, when a physically challenged or elderly person attempts to do an exercise with the wearable device 100 worn on their body, the wearable device 100 may provide an assistance force for assisting a physical movement during their exercise. In an example embodiment, the wearable device 100 may provide the assistance force and the resistance force in a combined way according to each exercise interval or time interval, for example, by providing the assistance force in an exercise interval and the resistance force in another exercise interval.

In an example embodiment, the wearable device 100 may operate in a physical ability measurement mode to measure a physical ability of the user 110. While the user 110 is walking or doing an exercise, the wearable device 100 may measure movement information of the user 110 using sensors (e.g., an angle sensor 125 and an inertial measurement unit (IMU) 135) provided in the wearable device 100 and evaluate a physical ability of the user 110 based on the measured movement information. For example, based on the movement information of the user 110 measured by the wearable device 100, a gait index or an exercise ability index (e.g., muscular strength, endurance, balance, and exercise posture) of the user 110 may be estimated. The physical ability measurement mode may include an exercise posture measurement mode for measuring an exercise posture performed by a user.

Although FIG. 1 illustrates an example of a hip-type wearable device for the convenience of description, a type of the wearable device 100 is not limited to the illustrated hip type. For example, the wearable device 100 may be provided in a type that is worn on other body parts (e.g., upper limbs, lower limbs, hands, calves, and feet) in addition to the waist and legs (thighs, in particular), and the shape and configuration of the wearable device 100 may vary according to a body part on which it is worn.

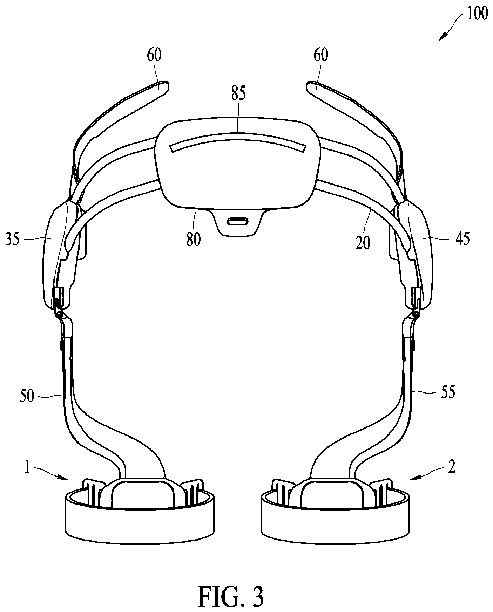

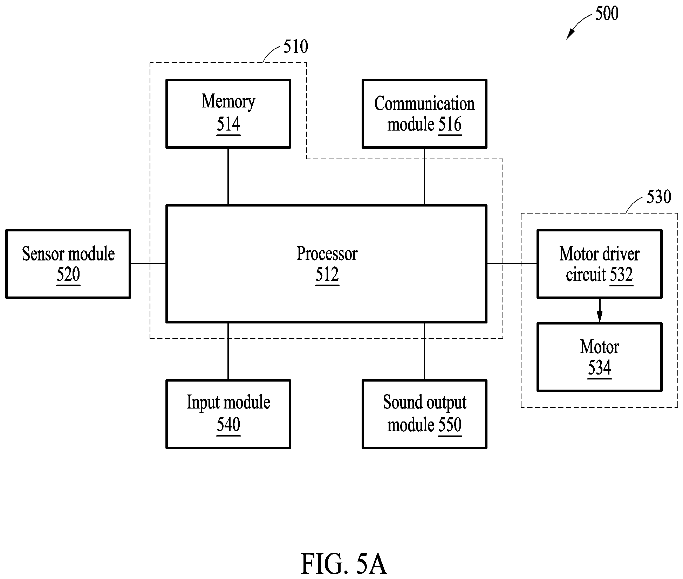

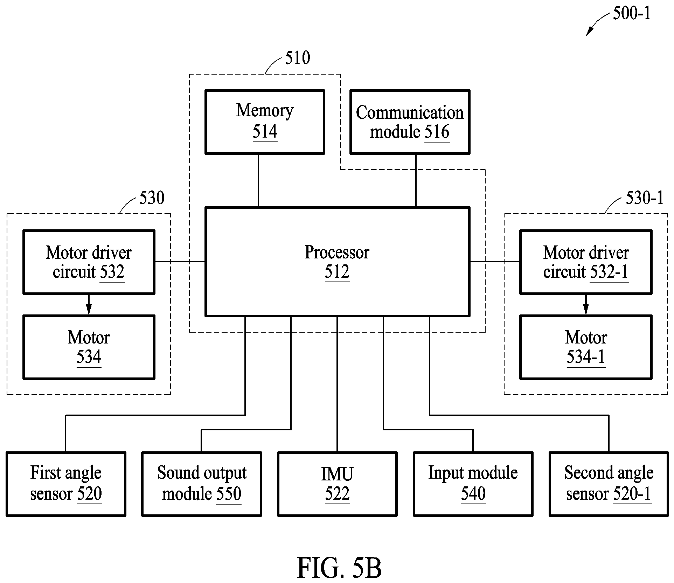

According to an example embodiment, the wearable device 100 may include a support frame (e.g., leg support frames 50 and 55 and a waist support frame 20 of FIG. 3) configured to support the body of the user 110 when the wearable device 100 is worn on the body of the user 110, a sensor module (e.g., a sensor module 520 of FIG. 5A, comprising at least one sensor) configured to obtain sensor data including movement information about a body movement (e.g., a movement of the legs and a movement of the upper body) of the user 110, the driving module 120 (e.g., driving modules 35 and 45 of FIG. 3) configured to generate torque to be applied to the legs of the user 110, and a control module 130 (e.g., a control module 510 of FIGS. 5A and 5B) configured to control the wearable device 100.

The sensor module may include the angle sensor 125 and the IMU 135. The angle sensor 125 may measure a rotation angle of the leg support frame of the wearable device 100 corresponding to a hip joint angle value of the user 110. The rotation angle of the leg support frame measured by the angle sensor 125 may be estimated as the hip joint angle value (or a leg angle value) of the user 110. The angle sensor 125 may include, for example, an encoder and/or a Hall sensor. In an example embodiment, the angle sensor 125 may be present near a right hip joint and a left hip joint of the user 110, respectively. The IMU 135 may include an acceleration sensor and/or an angular velocity sensor and may measure a change in acceleration and/or angular velocity according to a movement of the user 110. For example, the IMU 135 may measure an upper body movement value of the user 110 corresponding to a movement value of the waist support frame (or a base body, e.g., a base body 80 of FIG. 3) of the wearable device 100. The movement value of the waist support frame measured by the IMU 135 may be estimated as the upper body movement value of the user 110.

In an example embodiment, the control module 130 and the IMU 135 may be arranged in the base body (e.g., the base body 80 of FIG. 3) of the wearable device 100. The base body may be positioned on a lumbar portion (e.g., a waist portion) of the user 110 while the wearable device 100 is worn on the user 110. The base body may be formed on or attached to the outside of the waist support frame of the wearable device 100. The base body may be provided on the lumbar portion of the user 110 to provide a cushioning feeling to the waist of the user 110 and may support the waist of the user 110 along with the waist support frame.

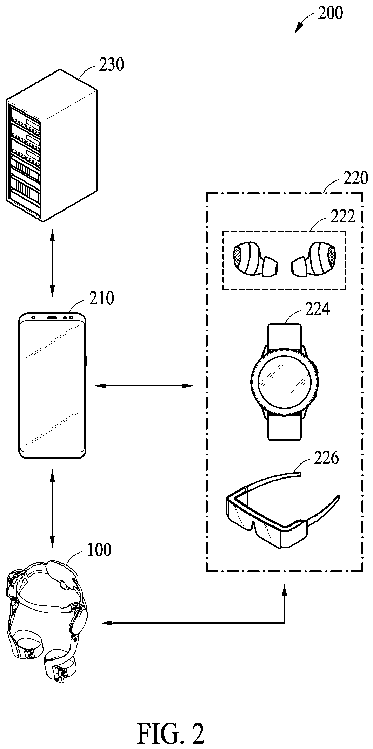

FIG. 2 is a diagram illustrating an example of an exercise management system including a wearable device and an electronic device according to an example embodiment.

Referring to FIG. 2, an exercise management system 200 may include a wearable device 100 to be worn on a body of a user, an electronic device 210, another wearable device 220, and a server 230. In an example embodiment, at least one (e.g., the other wearable device 220 or the server 230) of these may be omitted from the exercise management system 200 or at least one another device (e.g., a dedicated controller device of the wearable device 100) may be added to the exercise management system 200.

In an example embodiment, the wearable device 100 may be worn on the body of the user to assist the user with their movement in a walking assistance mode. For example, the wearable device 100 may be worn on legs of the user to generate an assistance force for assisting the user with a movement of the legs and assist the user in walking.

In an example embodiment, to enhance an exercise effect on the user in an exercise assistance mode, the wearable device 100 may generate a resistance force for hindering a physical movement of the user or an assistance force for assisting a physical movement of the user, and apply the generated resistance force or the generated assistance force to the body of the user. For example, in the exercise assistance mode, the user may select, through the electronic device 210, an exercise program (e.g., squat, split lunge, dumbbell squat, knee-up lunge, stretching, etc.) with which the user attempts to do an exercise using the wearable device 100, and/or an exercise intensity to be applied to the wearable device 100. The wearable device 100 may control a driving module of the wearable device 100 according to the exercise program selected by the user and obtain sensor data including movement information of the user through a sensor module comprising at least one sensor. The wearable device 100 may adjust the strength of the resistance force or the assistance force to be applied to the user according to the exercise intensity selected by the user. For example, the wearable device 100 may control the driving module to generate a resistance force corresponding to the exercise intensity selected by the user.

In an example embodiment, the wearable device 100 may be used to measure a physical ability of the user through interworking with the electronic device 210. The wearable device 100 may operate in a physical ability measurement mode which is a mode for measuring a physical ability of the user under the control of the electronic device 210 and may transmit sensor data obtained by a movement of the user in the physical ability measurement mode to electronic device 210. The electronic device 210 may then estimate the physical ability of the user by analyzing the sensor data received from the wearable device 100.

The electronic device 210 may communicate with the wearable device 100, and remotely control the wearable device 100 or provide the user with state information associated with a state (e.g., a booting state, a charging state, a sensing state, and an error state) of the wearable device 100. The electronic device 210 may receive the sensor data obtained by a sensor of the wearable device 100 from the wearable device 100 and estimate a physical ability of the user or a result of an exercise performed by the user based on the received sensor data. In an example embodiment, when the user is doing an exercise with the wearable device 100 worn on the user, the wearable device 100 may obtain sensor data including movement information of the user using sensors and transmit the obtained sensor data to the electronic device 210. The electronic device 210 may extract a movement value of the user from the sensor data and evaluate an exercise posture of the user based on the extracted movement value. The electronic device 210 may provide the user with an exercise posture measurement value and exercise posture evaluation information associated with the exercise posture of the user through a graphical user interface (GUI).

In an example embodiment, the electronic device 210 may execute a program (e.g., an application) for controlling the wearable device 100, and the user may adjust, through the program, an operation of the wearable device 100 or setting values (e.g., an intensity of torque output from the driving module (e.g., the driving modules 35 and 45 of FIG. 3, comprising driving circuitry), a size of audio output from a sound output module (e.g., a sound output module 550 of FIGS. 5A and 5B), and a brightness of a lighting unit (e.g., a lighting unit 85 of FIG. 3)). The program executed on the electronic device 210 may provide a GUI for an interaction with the user. The electronic device 210 may be a device in one of various type. The electronic device 210 may include, as non-limiting examples, a portable communication device (e.g., a smartphone), a computer device, an access point, a portable multimedia device, or a home appliance (e.g., a television (TV), an audio device, and a projector device).

According to an example embodiment, the electronic device 210 may be connected to the server 230 using short-range wireless communication or cellular communication. The server 230 may receive user profile information of the user using the wearable device 100 from the electronic device 210 and store and manage the received user profile information. The user profile information may include, for example, information about at least one of name, age, gender, height, weight, or body mass index (BMI) of the user. The server 230 may receive, from the electronic device 210, exercise history information about an exercise performed by the user, and store and manage the received exercise history information. The server 230 may provide the electronic device 210 with various exercise programs or physical ability measurement programs that may be provided to the user.

According to an example embodiment, the wearable device 100 and/or the electronic device 210 may be connected to the other wearable device 220. The other wearable device 220 may include, as non-limiting examples, wireless earphones 222, a smartwatch 224, or smart glasses 226. In an example embodiment, the smartwatch 224 may measure a biosignal including heart rate information of the user and transmit the measured biosignal to the electronic device 210 and/or the wearable device 100. The electronic device 210 may estimate the heart rate information (e.g., current heart rate, maximum heart rate, and average heart rate) of the user based on the biosignal received from the smartwatch 224 and provide the estimated heart rate information to the user.

In an example embodiment, the exercise result information, physical ability information, and/or exercise posture evaluation information that is evaluated through the electronic device 210 may be transmitted to the other wearable device 220 to be provided to the user through the other wearable device 220. The state information of the wearable device 100 may also be transmitted to the other wearable device 220 to be provided to the user through the other wearable device 220. In an example embodiment, the wearable device 100, the electronic device 210, and the other wearable device 220 may be connected to each other through wireless communication (e.g., Bluetooth communication and Wi-Fi communication).

In an example embodiment, the wearable device 100 may provide (or output) feedback (e.g., visual feedback, auditory feedback, and/or tactile feedback) corresponding to the state of the wearable device 100 according to a control signal received from the electronic device 210. For example, the wearable device 100 may provide visual feedback through the lighting unit (e.g., the lighting unit 85 of FIG. 3) and auditory feedback through the sound output module (e.g., the sound output module 550 of FIGS. 5A and 5B). The wearable device 100 may include a haptic module and provide tactile feedback in the form of vibration to the body of the user through the haptic module. The electronic device 210 may also provide (or output) feedback (e.g., visual feedback, auditory feedback, and/or tactile feedback) corresponding to the state of the wearable device 100.

In an example embodiment, the electronic device 210 may present a personalized exercise goal to the user in the exercise assistance mode. The personalized exercise goal may include a target exercise amount for each exercise type (e.g., a muscle strengthening exercise (or weight exercise), a balance exercise, an aerobic exercise (or cardio exercise)) that the user attempts to do, which may be determined by the electronic device 210 and/or the server 230. When the server determines the target exercise amount, the server 230 may transmit information about the determined target exercise amount to the electronic device 210. The electronic device 210 may then personalize and present a target exercise amount for an exercise type (e.g., the muscle strengthening exercise, the balance exercise, and the aerobic exercise) according to an exercise program (e.g., squat, split lunge, and knee-up lunge) the user attempts to perform and/or physical characteristics (e.g., age, height, weight, and BMI) of the user. The electronic device 210 may display, on a display, a GUI screen that displays the target exercise amount for each exercise type.

In an example embodiment, the electronic device 210 and/or the server 230 may include a database (DB) in which information about a plurality of exercise programs to be provided to the user through the wearable device 100 is stored. To achieve the exercise goal for the user, the electronic device 210 and/or the server 230 may recommend an exercise program that is suitable for the user. The exercise goal may include, for example, at least one of improving muscular strength, improving muscular physical strength, improving cardiovascular endurance, improving core stability, improving flexibility, or improving symmetry. The electronic device 210 and/or the server 230 may store and manage the exercise program performed by the user, a result of performing the exercise program, and the like.

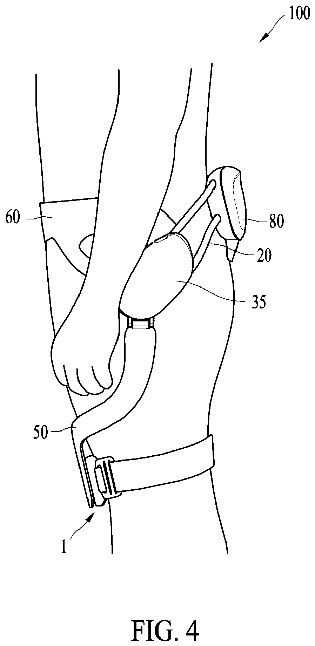

FIG. 3 is a rear view of an example of a wearable device according to an example embodiment, and FIG. 4 is a left side view of an example of a wearable device according to an example embodiment.

According to an example embodiment, referring to FIGS. 3 and 4, the wearable device 100 may include a base body 80, a waist support frame 20, driving modules 35 and 45, leg support frames 50 and 55, thigh fasteners 1 and 2, and a waist fastener 60. The base body 80 may include a lighting unit 85. In an example embodiment, at least one of these components (e.g., the lighting unit 85) may be omitted from or at least one other component (e.g., a haptic module) may be added to the wearable device 100.

The base body 80 may be positioned on the waist of the user while the wearable device 100 is worn on a body of a user. The base body 80 may be positioned on the waist of the user to provide a cushioning feeling to the waist of the user and support the waist of the user. The base body 80 may be hung around buttocks of the user such that the wearable device 100 does not escape downward by gravity while the wearable device 100 is worn on the user. The base body 80 may distribute a portion of the weight of the wearable device 100 to the waist of the user while the wearable device 100 is worn on the user. The base body 80 may be connected to the waist support frame 20. At both ends of the base body 80, a waist support frame connection element (not shown) that may be connected to the waist support frame 20 may be provided.

In an example embodiment, the lighting unit 85 may be disposed outside the base body 80. The lighting unit 85 may include a light source (e.g., a light-emitting diode (LED)). The lighting unit 85 may emit light under the control of a control module (not shown) (e.g., a control module 510 of FIGS. 5A and 5B). Depending on an example embodiment, the control module may control the lighting unit 85 to provide (or output) visual feedback corresponding to a state of the wearable device 100 to the user through the lighting unit 85.

The waist support frame 20 may extend from both ends of the base body 80. Inside the waist support frame 20, the waist of the user may be accommodated. The waist support frame 20 may include at least one rigid body beam. Each beam may be provided in a curved shape having a preset curvature to surround the waist of the user. The waist fastener 60 may be connected to an end of the waist support frame 20. The driving modules 35 and 45 may be connected to the waist support frame 20.

In an example embodiment, the control module (not shown), an IMU (not shown) (e.g., the IMU 135 of FIG. 1 and an IMU 522 of FIG. 5B), a communication module (not shown) (e.g., a communication module 516 of FIGS. 5A and 5B), and a battery (not shown) may be disposed inside the base body 80. The base body 80 may protect the control module, the IMU, the communication module, and the battery. The control module may generate a control signal for controlling an operation of the wearable device 100. The control module may include a control circuit including at least one processor and a memory to control actuators of the driving modules 35 and 45. The control module may further include a power supply module (not shown) to supply power of the battery to each of the components of the wearable device 100.

In an example embodiment, the wearable device 100 may include a sensor module (not shown) (e.g., a sensor module 520 of FIG. 5A) configured to obtain sensor data from at least one sensor. The sensor module may obtain the sensor data that changes according to a movement of the user. In an example embodiment, the sensor module may obtain the sensor data including movement information of the user and/or movement information of components of the wearable device 100. The sensor module may include, for example, an IMU (e.g., the IMU 135 of FIG. 1 or an IMU 522 of FIG. 5B) for measuring an upper body movement value of the user or a movement value of the waist support frame 20, and an angle sensor (e.g., the angle sensor 125 of FIG. 1, and a first angle sensor 520 and a second angle sensor 520-1 of FIG. 5B) for measuring a hip joint angle value of the user or a movement value of the leg support frames 50 and 55, but is not limited thereto. The sensor module may further include, for example, at least one of a position sensor, a temperature sensor, a biosignal sensor, or a proximity sensor.

The waist fastener 60 may be connected to the waist support frame 20 to fasten the waist support frame 20 to the waist of the user. The waist fastener 60 may include, for example, a pair of belts.

The driving modules 35 and 45 may generate an external force (or torque) to be applied to the body of the user based on the control signal generated by the control module. For example, the driving modules 35 and 45 may generate an assistance force or a resistance force to be applied to the legs of the user. In an example embodiment, the driving modules 35 and 45 may include a first driving module 45 disposed at a position corresponding to a position of a right hip joint of the user and a second driving module 35 disposed at a position corresponding to a position of a left hip joint of the user. The first driving module 45 may include a first actuator and a first joint member, and the second driving module 35 may include a second actuator and a second joint member. The first actuator may provide power to be transmitted to the first joint member, and the second actuator may provide power to be transmitted to the second joint member. The first actuator and the second actuator may each include a motor configured to generate power (or torque) by receiving power from the battery. When powered and driven, the motor may generate a force (e.g., the assistance force) for assisting a physical movement of the user or a force (e.g., the resistance force) for hindering a physical movement of the user. In an example embodiment, the control module may adjust a voltage and/or current to be supplied to the motor to adjust the intensity and direction of the force to be generated by the motor.

In an example embodiment, the first joint member and the second joint member may receive power from the first actuator and the second actuator, respectively, and may apply an external force to the body of the user based on the received power. The first joint member and the second joint member may be disposed at corresponding positions of joint portions of the user, respectively. One side of the first joint member may be connected to the first actuator, and the other side thereof may be connected to a first leg support frame 55. The first joint member may be rotated by the power received from the first actuator. An encoder or a Hall sensor that may operate as the angle sensor for measuring a rotation angle (corresponding to a joint angle of the user) of the first joint member may be disposed on one side of the first j oint member. One side of the second j oint member may be connected to the second actuator, and the other side thereof may be connected to a second leg support frame 50. The second joint member may be rotated by the power received from the second actuator. An encoder or a Hall sensor that may operate as the angle sensor for measuring a rotation angle (corresponding to a joint angle of the user) of the second joint member may be disposed on one side of the second joint member.

In an example embodiment, the first actuator may be disposed in a lateral direction of the first joint member, and the second actuator may be disposed in a lateral direction of the second joint member. A rotation axis of the first actuator and a rotation axis of the first joint member may be disposed to be separate from each other, and a rotation axis of the second actuator and a rotation axis of the second joint member may also be disposed to be separate from each other. However, examples are not limited thereto, and each actuator and each joint member may share a rotation axis. In an example embodiment, each actuator may be disposed to be separate from each joint member. In this case, the driving modules 35 and 45 may further include a power transmission module (not shown) configured to transmit power from the respective actuators to the respective j oint members. The power transmission module may be a rotary body (e.g., a gear), or a longitudinal member (e.g., a wire, a cable, a string, a spring, a belt, or a chain). However, the scope of examples is not limited to the foregoing positional relationship between the actuators and the joint members, and the foregoing power transmission structure.

In an example embodiment, the leg support frames 50 and 55 may support the legs (e.g., thighs) of the user when the wearable device 100 is worn on the legs of the user. For example, the leg support frames 50 and 55 may transmit power (e.g., torque) generated by the driving modules 35 and 45 to the thighs of the user, and the power may act as an external force to be applied to a movement of the legs of the user. As one end of the leg support frames 50 and 55 is connected to a joint member to be rotated and the other end of the leg support frames 50 and 55 is connected to thigh fasteners 1 and 2, the leg support frames 50 and 55 may transmit the power generated by the driving modules 35 and 45 to the thighs of the user while supporting the thighs of the user. For example, the leg support frames 50 and 55 may push or pull the thighs of the user. The leg support frames 50 and 55 may extend in a longitudinal direction of the thighs of the user. The leg support frames 50 and 55 may be bent to wrap at least a portion of the circumference of the thighs of the user. The leg support frames 50 and 55 may include the first leg support frame 55 for supporting the right leg of the user and the second leg support frame 50 for supporting the left leg of the user.

The thigh fasteners 1 and 2 may be connected to the leg support frames 50 and 55 and may fix the leg support frames 50 and 55 to the thighs of the user. The thigh fasteners 1 and 2 may include a first thigh fastener 2 for fixing the first leg support frame 55 to the right thigh of the user, and a second thigh fastener 1 for fixing the second leg support frame 50 to the left thigh of the user.

In an example embodiment, the first thigh fastener 2 may include a first cover, a first fastening frame, and a first strap. The second thigh fastener 1 may include a second cover, a second fastening frame, and a second strap. The first cover and the second cover may apply torque generated by the driving modules 35 and 45 to the thighs of the user. The first cover and the second cover may be disposed on one side of the thighs of the user to push or pull the thighs of the user. The first cover and the second cover may be disposed on a front surface of the thighs of the user. The first cover and the second cover may be disposed along a circumferential direction of the thighs of the user. The first cover and the second cover may extend to both sides around the other ends of the leg support frames 50 and 55 and may include curved surfaces corresponding to the thighs of the user. One ends of the first cover and the second cover may be connected to corresponding fastening frames, and the other ends thereof may be connected to corresponding straps.

For example, the first fastening frame and the second fastening frame may be disposed to surround at least a portion of the circumference of the thighs of the user to prevent or reduce the thighs of the user from escaping from the leg support frames 50 and 55. The first fastening frame may have a fastening structure that connects the first cover and the first strap, and the second fastening frame may have a fastening structure that connects the second cover and the second strap.

The first strap may surround a remaining portion of the circumference of the right thigh of the user that is not covered by the first cover and the first fastening frame, and the second strap may surround a remaining portion of the circumference of the left thigh of the user that is not covered by the second cover and the second fastening frame. The first strap and the second strap may each include, for example, an elastic material (e.g., a band).

FIGS. 5A and 5B are diagrams illustrating example configurations of a control system of a wearable device according to an example embodiment.

Referring to FIG. 5A, the wearable device 100 may be controlled by a control system 500. The control system 500 may include a control module 510 comprising control circuitry, a communication module 516 comprising communication circuitry, a sensor module 520 comprising at least one sensor, a driving module 530 comprising driving circuitry, an input module 540 comprising input circuitry, and a sound output module 550 comprising circuitry. In an example embodiment, at least one of these components (e.g., the sound output module 550) may be omitted or at least one other component (e.g., a haptic module) may be added to the control system 500.

The driving module 530 may include a motor 534 configured to generate power (e.g., torque) and a motor driver circuit 532 configured to drive the motor 534. Although a driving module (e.g., the driving module 530) is illustrated as including a single motor driver circuit (e.g., the motor driver circuit 532) and a single motor (e.g., the motor 534) in FIG. 5A, examples of which are not limited thereto. For example, as shown in FIG. 5B, a driving module (e.g., a driving module 530-1) of a control system 500-1 may include a plurality of (e.g., two or more) motor driver circuits (e.g., motor driver circuits 532 and 532-1) and motors (e.g., motors 534 and 534-1). The driving module 530 including the motor driver circuit 532 and the motor 534 may correspond to the first driving module 45 of FIG. 3, and the driving module 530-1 including the motor driver circuit 532-1 and the motor 534-1 may correspond to the second driving module 35 of FIG. 3. The following description of each of the motor driver circuit 532 and the motor 534 may also be applied to the motor driver circuit 532-1 and the motor 534-1 shown in FIG. 5B.

Referring back to FIG. 5A, the sensor module 520 may include a sensor circuit including at least one sensor. The sensor module 520 may include sensor data including movement information of a user or movement information of the wearable device 100. The sensor module 520 may transmit the obtained sensor data to the control module 510. The sensor module 520 may include an IMU 522 and an angle sensor (e.g., a first angle sensor 520 and a second angle sensor 520-1), as shown in FIG. 5B. The IMU 522 may measure an upper body movement value of the user. For example, the IMU 522 may sense X-axis, Y-axis, and Z-axis acceleration, and sense X-axis, Y-axis, and Z-axis angular velocity according to a movement of the user. The IMU 522 may be used to measure at least one of, for example, a forward and backward tilt, a leftward and rightward tilt, or a rotation of the body of the user. In addition, the IMU 522 may obtain a movement value (e.g., an acceleration value and an angular velocity value) of a waist support frame (e.g., the waist support frame 20 of FIG. 3) of the wearable device 100. The movement value of the waist support frame may correspond to the upper body movement value of the user.

The angle sensor may measure a hip joint angle value of the user according to a movement of the legs of the user. The sensor data that may be measured by the angle sensor may include, for example, a hip joint angle value of a right leg, a hip joint angle value of a left leg, and information about a direction of a movement of the legs. For example, the first angle sensor 520 of FIG. 5B may obtain the hip joint angle value of the right leg of the user, and the second angle sensor 520-1 may obtain the hip joint angle value of the left leg of the user. The first angle sensor 520 and the second angle sensor 520-1 may each include an encoder and/or a Hall sensor, for example. The angle sensor may also obtain a movement value of a leg support frame of the wearable device 100. For example, the first angle sensor 520 may obtain a movement value of the first leg support frame 55, and the second angle sensor 520-1 may obtain a movement value of the second leg support frame 50. The movement value of the leg support frame may correspond to the hip joint angle value.

In an example embodiment, the sensor module 520 may further include at least one of a position sensor for obtaining a position value of the wearable device 100, a proximity sensor for detecting proximity of an object, a biosignal sensor for detecting a biosignal of the user, or a temperature sensor for measuring an ambient temperature.

The input module 540 may receive a command or data to be used by a component (e.g., a processor 512) of the wearable device 100 from the outside (e.g., the user) of the wearable device 100. The input module 540 may include an input component circuit. The input module 540 may include, for example, a key (e.g., a button) or a touchscreen.

The sound output module 550 may output a sound signal to the outside of the wearable device 100. The sound output module 550 may provide auditory feedback to the user. For example, the sound output module 550 may include a speaker that reproduces a guide voice for an audible notification of a guide sound signal (e.g., a driving start sound, a posture error notification sound, or an exercise start notification sound), music content, or specific information (e.g., exercise result information and exercise posture evaluation information).

In an example embodiment, the control system 500 may further include a battery (not shown) for supplying power to each component of the wearable device 100. The wearable device 100 may convert power of the battery according to an operating voltage of each component of the wearable device 100 and supply the converted power to each component.

The driving module 530 may generate an external force to be applied to the legs of the user under the control of the control module 510. The driving module 530 may generate torque to be applied to the legs of the user based on a control signal generated by the control module 510. The control module 510 may transmit the control signal to the motor driver circuit 532. The motor driver circuit 532 may control an operation of the motor 534 by generating a current signal (or a voltage signal) corresponding to the control signal and supplying the generated current signal to the motor 534. As circumstances require, the current signal may not be supplied to the motor 534. When the motor 534 is driven as the current signal is supplied to the motor 534, the motor 534 may generate torque for an assistance force for assisting a movement of the legs of the user or a resistance force for hindering a movement of the legs of the user.

The control module 510 may control an overall operation of the wearable device 100 and may generate a control signal for controlling each component (e.g., the communication module 516 and the driving module 530). The control module 510 may include at least one processor 512 and a memory 514.

For example, the at least one processor 512 may execute software to control at least one other component (e.g., a hardware or software component) of the wearable device 100 connected to the processor 512 and may perform various types of data processing or computation. The software may include an application for providing a GUI. According to an example embodiment, as at least a part of data processing or computation, the processor 512 may store instructions or data received from another component (e.g., the communication module 516) in the memory 514, process the instructions or data stored in the memory 514, and store resulting data obtained by the processing in the memory 514. According to an example embodiment, the processor 512 may include, for example, a main processor (e.g., a central processing unit (CPU) or an application processor (AP)) or an auxiliary processor (e.g., a graphics processing unit (GPU), a neural processing unit (NPU), an image signal processor (ISP), a sensor hub processor, or a communication processor (CP)) that is operable independently of, or in conjunction with, the main processor. The auxiliary processor may be implemented separately from the main processor or as a part of the main processor. Each "processor" herein comprises processing circuitry.

The memory 514 may store various pieces of data used by at least one component (e.g., the processor 512) of the control module 510. The data may include, for example, input data or output data for software, sensor data, and instructions related thereto. The memory 514 may include a volatile memory or a non-volatile memory (e.g., a random-access memory (RAM), a dynamic RAM (DRAM), or a static RAM (SRAM)).

The communication module 516 may support establishment of a direct (e.g., wired) communication channel or a wireless communication channel between the control module 510 and another component of the wearable device 100 or an external electronic device (e.g., the electronic device 210 or the other wearable device 220 of FIG. 2), and support communication through the established communication channel. The communication module 516 may include a communication circuit for performing a communication function. For example, the communication module 516 may receive a control signal from an electronic device (e.g., the electronic device 210) and transmit the sensor data obtained by the sensor module 520 to the electronic device. The communication module 516 may include at least one CP (not shown) that is operable independently of the processor 512 and that supports the direct (e.g., wired) communication or the wireless communication. According to an example embodiment, the communication module 516 may include a wireless communication module (e.g., a cellular communication module, a short-range wireless communication module, or a global navigation satellite system (GNSS) communication module), and/or a wired communication module. A corresponding one of these communication modules may communicate with another component of the wearable device 100 and/or an external electronic device via a short-range communication network (e.g., Bluetooth^{™}, wireless-fidelity (Wi-Fi), or infrared data association (IrDA)), or a long-range communication network (e.g., a legacy cellular network, a 5G network, a next-generation communication network, the Internet, or a computer network (e.g., a local area network (LAN) or a wide area network (WAN)).

In an example embodiment, the control system (e.g., the control systems 500 and 500-1) may further include a haptic module (not shown). The haptic module may provide tactile feedback to the user under the control of the processor 512. The haptic module may convert an electrical signal into a mechanical stimulus (e.g., a vibration or a movement) or an electrical stimulus that may be recognized by the user via their tactile sensation or kinesthetic sensation. The haptic module may include, for example, a motor, a piezoelectric element, or an electrical stimulation device. In an example embodiment, the haptic module may be positioned on at least one of a base body (e.g., the base body 80), a first thigh fastener (e.g., the first thigh fastener 2), or a second thigh fastener (e.g., the second thigh fastener 1).

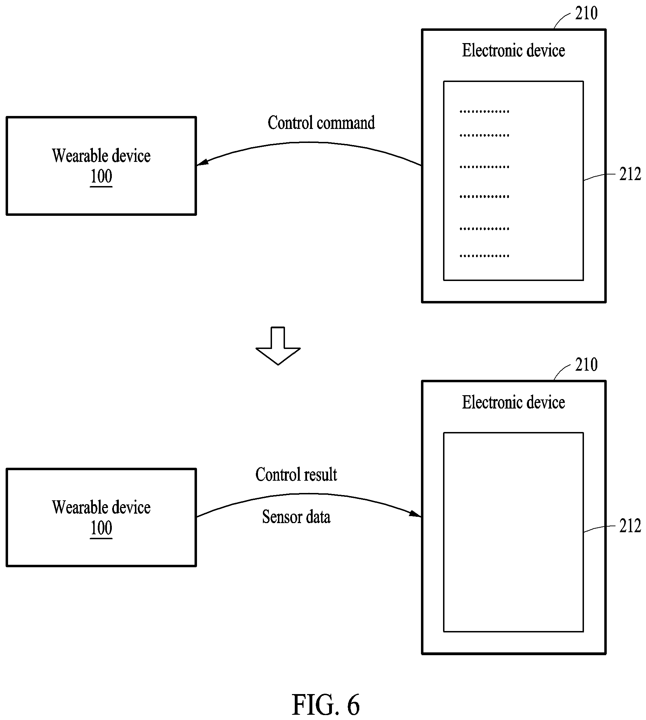

FIG. 6 is a diagram illustrating an example of an interaction between a wearable device and an electronic device according to an example embodiment.

Referring to FIG. 6, the wearable device 100 may communicate with the electronic device 210. For example, the electronic device 210 may be a user terminal of a user who uses the wearable device 100, or a dedicated controller for the wearable device 100. According to an example embodiment, the wearable device 100 and the electronic device 210 may be connected to each other through short-range wireless communication (e.g., Bluetooth communication or Wi-Fi communication).

In an example embodiment, the electronic device 210 may execute an application for checking a state of the wearable device 100 or controlling or operating the wearable device 100. When the application is executed, a screen of a user interface (UI) for controlling an operation of the wearable device 100 or determining an operation mode of the wearable device 100 may be displayed on a display 212 of the electronic device 210. The UI may be a graphical user interface (GUI), for example.

In an example embodiment, the user may input a command (e.g., a command for executing a walking assistance mode, an exercise assistance mode, or a physical ability measurement mode) for controlling the operation of the wearable device 100 or change settings of the wearable device 100, through the screen of the GUI on the display 212 of the electronic device 210. The electronic device 210 may generate a control command (or a control signal) corresponding to an operation control command or a settings change command that is input by the user and transmit the generated control command to the wearable device 100. The wearable device 100 may operate according to the received control command and may transmit, to the electronic device 210, a control result obtained in response to the control command and/or sensor data measured by the sensor module of the wearable device 100. The electronic device 210 may provide, to the user through the screen of the GUI, resulting information (e.g., walking ability information, exercise ability information, and exercise posture evaluation information) derived by analyzing the control result and/or the sensor data.

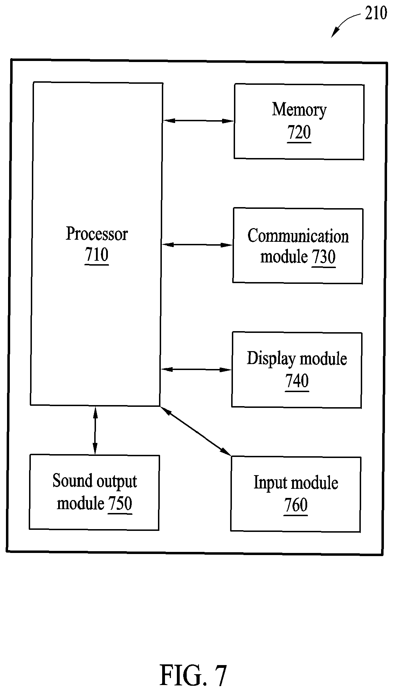

FIG. 7 is a diagram illustrating an example configuration of an electronic device according to an example embodiment.

Referring to FIG. 7, the electronic device 210 may include a processor 710, a memory 720, a communication module 730, a display module 740, a sound output module 750, and an input module 760. In an example embodiment, at least one of these components (e.g., the sound output module 750) may be omitted from or at least one other component (e.g., a sensor module and a battery) may be added to the electronic device 210.

The processor 710 may control at least one other component (e.g., a hardware or software component) of the electronic device 210 and may perform various types of data processing or computation. According to an example embodiment, as at least a part of data processing or computation, the processor 710 may store instructions or data received from another component (e.g., the communication module 730) in the memory 720, process the instructions or data stored in the memory 720, and store resulting data in the memory 720.

The processor 710 may include, for example, a main processor (e.g., a CPU or an AP) or an auxiliary processor (e.g., a GPU, a NPU, an ISP, a sensor hub processor, or a CP) that is operable independently of, or in conjunction with, the main processor.

The memory 720 may store various pieces of data used by at least one component (e.g., the processor 710 or the communication module 730) of the electronic device 210. The data may include, for example, input data or output data for a program (e.g., an application) and instructions related thereto. The memory 720 may include at least one instruction executable by the processor 710. The memory 720 may include a volatile memory or a non-volatile memory.

The communication module 730 may support the establishment of a direct (e.g., wired) communication channel or a wireless communication channel between the electronic device 210 and another electronic device (e.g., the wearable device 100, the other wearable device 220, and the server 230 as shown in Fig. 2 for example), and support the communication via the established communication channel. The communication module 730 may include a communication circuit for performing a communication function. The communication module 730 may include at least one communication processor (CP) that is operable independently of the processor 710 (e.g., an AP) and support direct (e.g., wired) communication or wireless communication. According to an example embodiment, the communication module 730 may include a wireless communication module (e.g., a Bluetooth communication module, a cellular communication module, a Wi-Fi communication module, or a GNSS communication module) or a wired communication module (e.g., a LAN communication module or a power line communication module). For example, the communication module 730 may transmit a control command to the wearable device 100 and receive at least one of sensor data including physical movement information of a user wearing the wearable device 100, state data of the wearable device 100, or control result data corresponding to the control command.

The display module 740 may visually provide information to the outside (e.g., the user) of the electronic device 210. The display module 740 may include, for example, a liquid-crystal display (LCD) or an organic light-emitting diode (OLED) display, a hologram device, or a projector device. The display module 740 may further include a control circuit for controlling driving of a display. In an example embodiment, the display module 740 may include a touch sensor configured to sense a touch, or a pressure sensor configured to measure an intensity of a force incurred by the touch.

The sound output module 750 may output a sound signal to the outside of the electronic device 210. The sound output module 750 may include a speaker that reproduces a guide sound signal (e.g., a driving start sound and an operation error notification sound), music content, or a guide voice based on a state of the wearable device 100. For example, in response to a determination that the wearable device 100 is not correctly worn on the body of the user, the sound output module 750 may output a guide voice to inform the user of the incorrect wearing or guide the user through normal wearing. For example, the sound output module 750 may output a guide voice corresponding to exercise evaluation information or exercise result information that is obtained by evaluating an exercise performed by the user.

The input module 760 may receive a command or data to be used by a component (e.g., the processor 710) of the electronic device 210 from the outside (e.g., the user) of the electronic device 210. The input module 760 may include an input component circuit and receive a user input. The input module 760 may include, for example, a key (e.g., a button) or a touchscreen.

According to an example embodiment, when the user performs an exercise (e.g., squat, split lunge, dumbbell squat, and knee-up lunge) that requires the repetition of exercise postures of the same type, the electronic device 210 may sense in real time a posture performed by the user through the wearable device 100. The electronic device 210 may analyze movement information (e.g., an upper body tilt value and a hip joint angle value) of the user obtained by at least one sensor of the wearable device 100 and provide exercise posture evaluation information and/or feedback information for correcting an exercise posture to the user through a GUI. The exercise posture evaluation information may include, for example, an evaluation level (e.g., great, good, and bad) of the exercise posture of the user and information about a measurement value of each evaluation element (e.g., an upper body tilt value and a hip joint angle difference between both hip joints). The feedback information for correcting the exercise posture may include, for example, information about how to correct the exercise posture for each evaluation element to improve the evaluation level of the exercise posture of the user. Through the wearable device 100 and the electronic device 210, the user may receive a result of determining whether the exercise posture of the user is appropriate without the help of an expert and receive an accurate measurement value of the exercise posture performed by the user. The user may then clearly recognize a desirable exercise posture and effectively correct the exercise posture, based on the exercise posture evaluation information and/or the feedback information provided through the electronic device 210.

In an example embodiment, the electronic device 210 may include the input module 760 configured to receive a user input that selects an exercise program to be performed by the user wearing the wearable device 100, the communication module 730 configured to receive sensor data including movement information of the user during an exercise performed by the user according to the selected exercise program, the processor 710 configured to generate a visual guide object indicating evaluation information associated with an exercise posture of the user by analyzing the sensor data, and the display module 740 configured to output a GUI on which the visual guide object is displayed.

In an example embodiment, the user may select an exercise program and/or exercise intensity the user desires to perform, through the input module 760. When the user selects the exercise program and/or exercise intensity, the processor 710 may provide a personalized target exercise amount for the user. When the user inputs an exercise start command through the GUI after preparing to start the exercise, the processor 710 may control the communication module 730 to transmit, to the wearable device 100, a control command for requesting the start of measurement of an exercise posture of the user.

After the user starts the exercise, the communication module 730 may receive, from the wearable device 100, sensor data including at least one of an upper body movement value corresponding to a movement of an upper body of the user or a hip joint angle value corresponding to a movement of legs of the user. The processor 710 may determine an exercise posture measurement value of the user based on the received sensor data. Based on the sensor data, the processor 710 may determine the exercise exposure measurement value including at least one of an upper body tilt value of the user or a hip joint angle difference value between both hip joints of the user. For example, the processor 710 may determine, to be the exercise posture measurement value, at least one of a maximum or high upper body tilt value of the user or a maximum or high hip joint angle difference value between both hip joints that is measured from a previous exercise posture of the user. A type of the exercise posture measurement value determined by the processor 710 may not be limited to the foregoing examples, and the processor 710 may determine an exercise posture measurement value corresponding to at least one exercise posture evaluation element defined in the exercise program performed by the user.

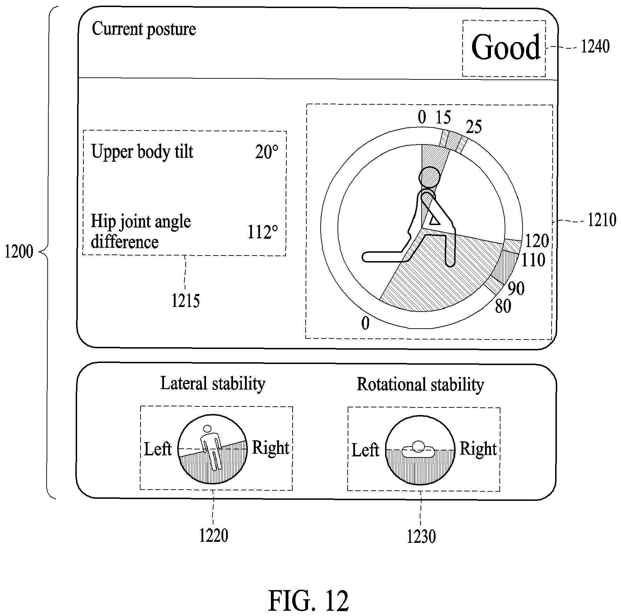

In an example embodiment, the processor 710 may generate a first visual guide object (e.g., a first visual object 1210 of FIG. 12) including a first indicator that indicates an exercise posture reference for the exercise program selected by the user and at least one second indicator that indicates the determined exercise posture measurement value. The display module 740 may output a GUI on which the first visual guide object is displayed. The display module 740 may visualize and provide feedback on an exercise posture of the user through the GUI.

In an example embodiment, the processor 710 may evaluate the exercise posture of the user based on an exercise posture reference representing a standard angle value for each exercise posture. The exercise posture reference may be indicated by a physical movement value for a standard posture indicated for each exercise program. The memory 720 may store information about an exercise posture reference for each exercise program. The processor 710 may display an exercise posture measurement value of the user with respect to the exercise posture reference as a graphical element such as, for example, graphs and icons, through the first visual guide object. In an example embodiment, the first visual guide object may be used to effectively provide the user with an upper body tilt value and a hip joint angle difference value between both hip joints that are obtained from a current exercise posture of the user, along with an upper body tilt value and a hip j oint angle difference value for a desirable exercise posture. Through the first visual guide object, the user may easily recognize which body part of them needs to be corrected and how to correct the body part to perform the desirable exercise posture.

In an example embodiment, the first indicator of the first visual guide object may indicate a plurality of exercise posture reference ranges corresponding to different exercise posture evaluation levels. The at least one second indicator may include a second indicator that indicates an exercise posture measurement value based on a previous exercise posture of the user or a second indicator that indicates in real time an exercise posture measurement value based on a current exercise posture of the user. For example, the second indicator indicating the exercise posture measurement value based on the previous exercise posture may indicate at least one of a maximum or high upper body tilt value based on the previous exercise posture of the user or a maximum or high hip joint angle difference value between both hip joints of the user based on the previous exercise posture of the user.

In an example embodiment, the at least one second indicator may have a color to be displayed in the first visual guide object that varies according to an evaluation reference range to which the exercise posture measurement value indicated by the at least one second indicator belongs.

In an example embodiment, the first indicator may indicate an exercise posture reference range for the exercise program selected by the user, and the at least one second indicator may indicate in real time an exercise posture measurement value of the user. When the exercise posture measurement value is included in the exercise posture reference range indicated by the first indicator, the at least one second indicator may change to a color corresponding to the exercise posture reference range.

In an example embodiment, the first visual guide object may further include at least one third indicator that indicates an angle range indicated by a fixed reference line and an angle line that varies in indication according to an exercise posture measurement value of the user. The at least one third indicator may have a color to be displayed in the first visual guide object that varies according to the evaluation reference range to which the exercise posture measurement value belongs. In an example embodiment, the at least one third indicator may include a third indicator that indicates an angle range indicated by a reference line for an upper body tilt value of the user and an angle line corresponding to a maximum or high upper body tilt value of the user determined from a previous exercise posture of the user, or a third indicator that indicates an angle range indicated by a reference line for a hip joint angle difference value between both hip joints of the user and an angle line corresponding to a maximum or high hip joint angle difference value between both hip joints of the user determined from the previous exercise posture of the user.

In an example embodiment, the first visual guide object may further include an avatar object having a shape corresponding to a representative exercise posture of the exercise program selected by the user. In an example embodiment, the avatar object may be displayed at a center of the first visual guide object, and the at least one third indicator may be displayed as being overlaid on the avatar object. In an example embodiment, the avatar object may have a posture to be displayed that changes according to an exercise posture of the user. For example, when the exercise posture of the user changes during the exercise, the avatar object may change in shape according to the changed exercise posture of the user. In an example embodiment, the avatar object may be used to provide exercise posture evaluation information determined for each exercise posture evaluation reference for the user. In each body part area of the avatar object, the exercise posture evaluation information of each body part of the user may be provided through graphic elements.

In an example embodiment, the processor 710 may determine an exercise posture measurement value corresponding to physical movement stability of the user based on the sensor data received from the wearable device 100. The processor 710 may generate at least one second visual guide object (e.g., second visual guide objects 1220 and 1230 of FIG. 12) that indicates the determined physical movement stability. The display module 740 may output a GUI on which the second visual guide object is displayed. In an example embodiment, the second visual guide object may be provided through one screen of the GUI along with the first visual guide object.

In an example embodiment, the at least one second visual guide object may include a second visual guide object (e.g., the second visual guide object 1220) that indicates lateral (or left-right) stability of a physical movement of the user or a second visual guide object (e.g., the second visual guide object 1230) that indicates rotational stability of the physical movement of the user.

In an example embodiment, the at least one second visual guide object may include a fixed reference line, an avatar object of which a tilt to be displayed changes according to an exercise posture measurement value corresponding to the physical movement stability, and a color object of which a color changes according to an exercise posture evaluation level of the exercise posture measurement value corresponding to the physical movement stability, but examples of which are not limited thereto.

In an example embodiment, the processor 710 may control the communication module 730 to transmit the exercise posture evaluation information of the user to the wearable device 100, thereby allowing the wearable device 100 to provide a feedback signal corresponding to the exercise posture evaluation information to the user. For example, when the exercise program requires the repetition of the same exercise posture, the processor 710 may determine exercise posture evaluation information associated with an exercise posture each time the user completes each exercise posture, and may control the communication module 730 to transmit a feedback signal corresponding to the determined exercise posture evaluation information to the wearable device 100. The wearable device 100 may output a guide voice for the feedback signal received from the electronic device 210 through the sound output module (e.g., the sound output module 550 of FIGS. 5A and 5B) whenever each exercise posture is completed. For example, the wearable device 100 may output a guide voice corresponding to any one of "great," "good," and "bad" as an evaluation of an exercise posture of the user, or may output a guide voice relating to the correction of the exercise posture.

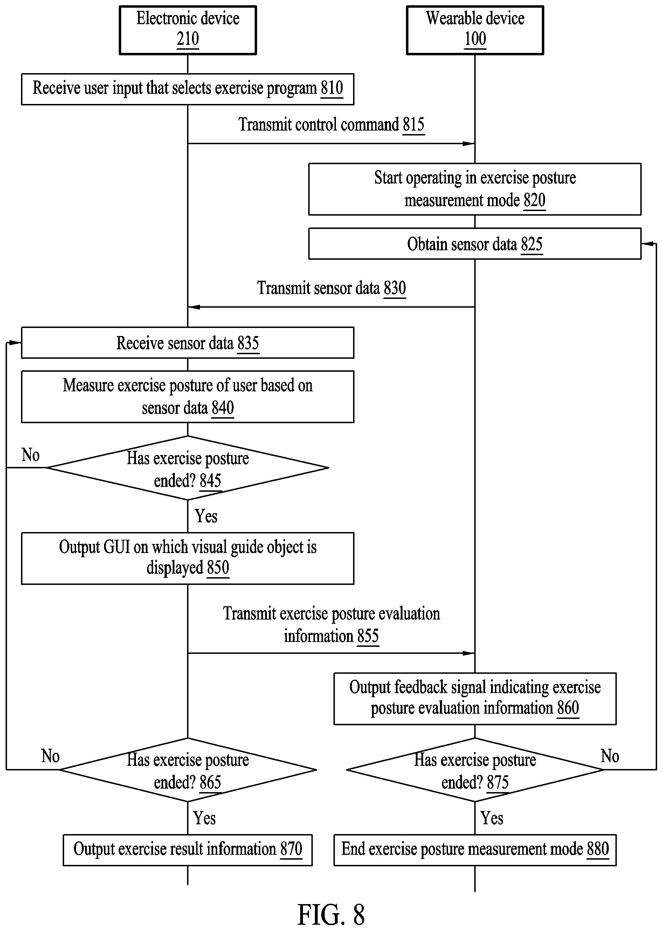

FIG. 8 is a flowchart illustrating an example of an operation method of an electronic device and a wearable device according to an example embodiment. In an example embodiment, at least one of operations to be described below with reference to FIG. 8 may be performed simultaneously or in parallel with another operation, and the order of the operations may be changed. In addition, at least one of the operations may be omitted, or another operation may be added.

Referring to FIG. 8, in operation 810, the electronic device 210 may receive a user input that selects an exercise program to be performed by a user wearing the wearable device 100. In an example embodiment, the electronic device 210 may recommend exercise programs suitable for a physical characteristic of the user, and the user may select an exercise program (e.g., squat, half squat, stretching, split lunge, dumbbell squat, and knee-up lunge) to be performed by the user from a list of the recommended exercise programs provided through a GUI. The electronic device 210 may provide the user with a GUI for controlling and setting the wearable device 100 through a program (e.g., an application).

In operation 815, when the user completes selecting the exercise program and setting details of an exercise (e.g., an exercise time, the number of repetitions of an exercise posture, and an exercise intensity) through the GUI, the electronic device 210 may notify the wearable device 100 of the start of the exercise of the user and transmit a control command for requesting measurement of an exercise posture of the user to the wearable device 100. In this case, setting data of the details of the exercise set for the exercise program by the user may be included in the control command to be transmitted to the wearable device 100.

In operation 820, the wearable device 100 may receive the control command from the electronic device 210 and, in response to a verification that the user is wearing the wearable device 100, may start operating in an exercise posture measurement mode for measuring an exercise posture of the user.

In operation 825, the wearable device 100 may obtain sensor data including movement information according to a movement of the user during the exercise, using a sensor module. For example, the wearable device 100 may obtain an upper body tilt value of the user using an IMU and obtain a hip joint angle value of each of both hip joints of the user using an angle sensor. In an example embodiment, the wearable device 100 may extract a maximum or high value among upper body tilt values measured in a process in which the user performs exercise postures once, and extract a maximum or high value among hip joint angle values of each of both hip joints measured in the process in which the user performs the exercise postures once. The wearable device 100 may measure a movement value for at least one of a forward and backward tilt, a leftward and rightward tilt, or a rotation of the upper body of the user, using the IMU.

In operation 830, the wearable device 100 may transmit the obtained sensor data to the electronic device 210. For example, the wearable device 100 may transmit, to the electronic device 210, the maximum value of the upper body tilt values measured during one time of performing the exercise postures and the maximum value of the hip joint angle values of both hip joints measured during one time of performing the exercise postures. In an example embodiment, the wearable device 100 may transmit the sensor data to the electronic device 210 in real time or on a periodic basis during the exercise performed by the user.

In operation 835, the electronic device 210 may receive, from the wearable device 100, the sensor data including the movement information of the user during the exercise performed according to the exercise program.

In operation 840, the electronic device 210 may measure an exercise posture of the user based on the sensor data. The electronic device 210 may determine an exercise posture measurement value of the user based on the sensor data. The electronic device 210 may determine the exercise posture measurement value including at least one of an upper body tilt value of the user or a hip joint angle difference value between both hip joints of the user based on the sensor data. For example, the electronic device 210 may determine, as the exercise posture measurement value, at least one of a maximum upper body tilt value of the user measured from a previous exercise posture of the user or a maximum hip joint angle difference value between both hip joints measured from the previous exercise posture.

In operation 845, the electronic device 210 may determine whether the exercise posture of the user ends. In an example embodiment, the electronic device 210 may determine whether the exercise posture of the user has ended based on a pattern in the sensor data. For example, when it is estimated that the user is taking a basic posture (e.g., a standing posture) indicated in the exercise program or when no physical movement value of the user is detected for a predetermined period of time, as a result of analyzing the sensor data, the electronic device 210 may determine that the user has ended the exercise posture.

When it is determined that the exercise posture of the user has not ended (No in operation 845), the electronic device 210 may perform again an operation of determining an exercise posture measurement value of the user based on the sensor data.

In operation 850, when it is determined that the exercise posture of the user has ended (Yes in operation 845), the electronic device 210 may evaluate the exercise posture of the user based on an exercise posture reference for each exercise program, and output a GUI on which a visual guide object indicating exercise posture evaluation information is displayed. The electronic device 210 may compare the exercise posture reference and the measured exercise posture of the user, and determine the exercise posture evaluation information of the user based on a result of the comparison. The electronic device 210 may visualize and visually display feedback on the exercise posture of the user through the visual guide object.

In an example embodiment, the electronic device 210 may output a GUI on which a first visual guide object including a first indicator indicating an exercise posture reference for the exercise program selected by the user and at least one second indicator indicating a determined exercise posture measurement value is displayed. The first visual guide object may further include at least one third indicator indicating an angle range defined by a fixed reference line and an angle line of which an indication changes according to an exercise posture measurement value of the user. The electronic device 210 may determine an exercise posture measurement value corresponding to physical movement stability of the user based on the sensor data received from the wearable device 100, and output a GUI on which at least one second visual guide object indicating the determined physical movement stability is displayed. The first visual guide object and the second visual guide object may be provided together through one screen of the GUI or separately provided through different screens.

In operation 855, the electronic device 210 may transmit the exercise posture evaluation information of the user to the wearable device 100.

In operation 860, when receiving the exercise posture evaluation information from the electronic device 210, the wearable device 100 may output a feedback signal indicating the exercise posture evaluation information. The wearable device 100 may provide the user with evaluation feedback on the exercise posture of the user based on the feedback signal. For example, the wearable device 100 may output a guide voice for the feedback signal through a sound output module.

In operation 865, the electronic device 210 may determine whether the exercise of the user ends. In an example embodiment, in at least one of a case in which the number of repetitions of exercise postures of the user reaches an end time, a case in which a predefined time has elapsed, or a case in which an end command for ending the exercise posture measurement mode is received through a user input, the electronic device 210 may determine that the exercise of the user has ended.

When it is determined that the exercise of the user has not ended (No in operation 865), the electronic device 210 may perform the operations again on an exercise posture of a subsequent time, starting from operation 835. In operation 870, when it is determined that the exercise of the user has ended (Yes in operation 865), the electronic device 210 may output, through a GUI, exercise result information obtained by performing the exercise program by the user. The exercise result information may include, for example, information about a total calorie consumption value which is a value of calories consumed by the exercise, an exercise execution time, and a final evaluation of an exercise posture.

In operation 875, the wearable device 100 may determine whether the exercise of the user ends. In an example embodiment, when receiving the end command for ending the exercise posture measurement mode through the electronic device 210 or a user input, the wearable device 100 may determine that the exercise of the user has ended. When it is determined that the exercise has not ended (No in operation 875), the wearable device 100 may perform the operations again, starting from operation 825 of obtaining sensor data of an exercise posture of a subsequent time. In operation 880, when it is determined that the exercise has ended (Yes in operation 875), the wearable device 100 may end the exercise posture measurement mode. When operations in the exercise posture measurement mode have ended, the wearable device 100 may enter a wait mode.

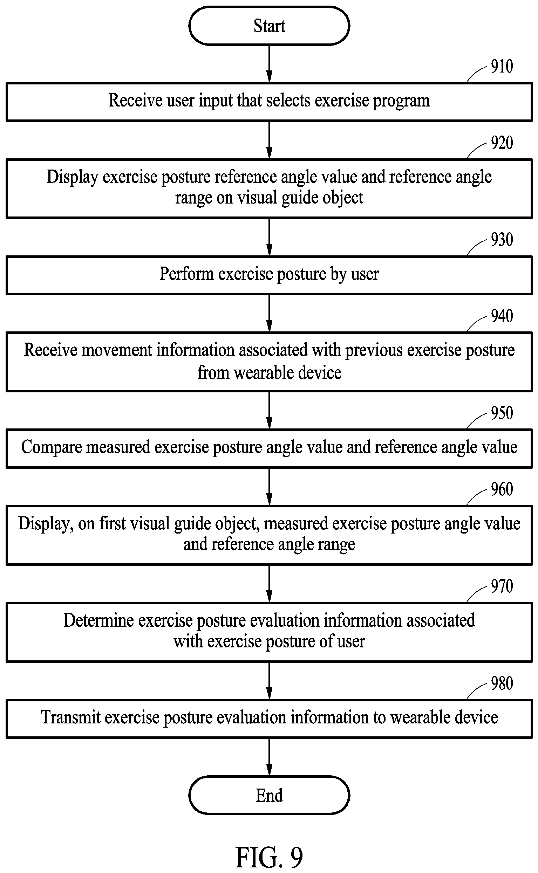

FIG. 9 is a flowchart illustrating an example of a method of providing exercise posture evaluation information of a user according to an example embodiment. In an example embodiment, at least one of operations to be described below with reference to FIG. 9 may be performed simultaneously or in parallel with another operation, and the order of the operations may be changed. In addition, at least one of the operations may be omitted, or another operation may be added.

Referring to FIG. 9, in operation 910, the electronic device 210 may receive a user input that selects an exercise program to be performed by a user.

In operation 920, the electronic device 210 may display a reference angle value and a reference angle range of an exercise posture to be evaluated in the exercise program selected by the user, using first indicators of a first visual guide object displayed on a GUI. The reference angle value and the reference angle range may indicate respectively a desirable angle value and a desirable angle range of a body (e.g., an upper body and legs) that need to be achieved by the user in a process of performing exercise postures.

In operation 930, the user may perform the exercise posture according to the exercise program with the wearable device 100 worn on the body of the user. The wearable device 100 may obtain sensor data including movement information of the user through sensors in the process of performing the exercise postures, and transmit the obtained sensor data to the electronic device 210. For example, the wearable device 100 may transmit, to the electronic device 210, a maximum upper body tilt value and a maximum hip joint angle value of each of both hip j oints that are measured in the process of performing the exercise postures once by the user.

In operation 940, the electronic device 210 may receive movement information associated with a previous exercise posture from the wearable device 100. In operation 950, the electronic device 210 may compare, to the reference angle value, an angle value of the exercise posture of the user measured from the received movement information.

In operation 960, the electronic device 210 may display, on the first visual guide object, the measured angle value of the exercise posture and the reference angle range. In an example embodiment, the electronic device 210 may display, on the first visual guide object, a first indicator that indicates a plurality of exercise posture reference ranges corresponding to different exercise posture evaluation levels, and a second indicator that indicates an exercise posture measurement value (e.g., a maximum upper body tilt value and a maximum hip joint angle difference value between both hip joints) that is measured from an immediately previous exercise posture of the user. The electronic device 210 may further display, on the first visual guide object, at least one third indicator that indicates an angle range defined by a fixed reference line and an angle line of which an indication changes according to the exercise posture measurement value of the user. In addition, the electronic device 210 may output at least one second visual guide object that indicates physical movement stability evaluated in a process of performing the previous exercise posture by the user.

In operation 970, the electronic device 210 may determine exercise posture evaluation information associated with the exercise posture of the user. The electronic device 210 may evaluate the exercise posture of the user by comparing the reference angle value defined for each exercise program and the angle value of the exercise posture of the user measured from the movement information of the user. The electronic device 210 may determine a final evaluation level (e.g., great, good, or bad) of the immediately previous exercise posture of the user based on the exercise posture reference ranges.

In operation 980, the electronic device 210 may transmit the exercise posture evaluation information to the wearable device 100. The wearable device 100 may output a feedback signal for notifying the user of the exercise posture evaluation information based on the received exercise posture evaluation information. For example, the wearable device 100 may output a guide voice for the final evaluation level of the previous exercise posture of the user.

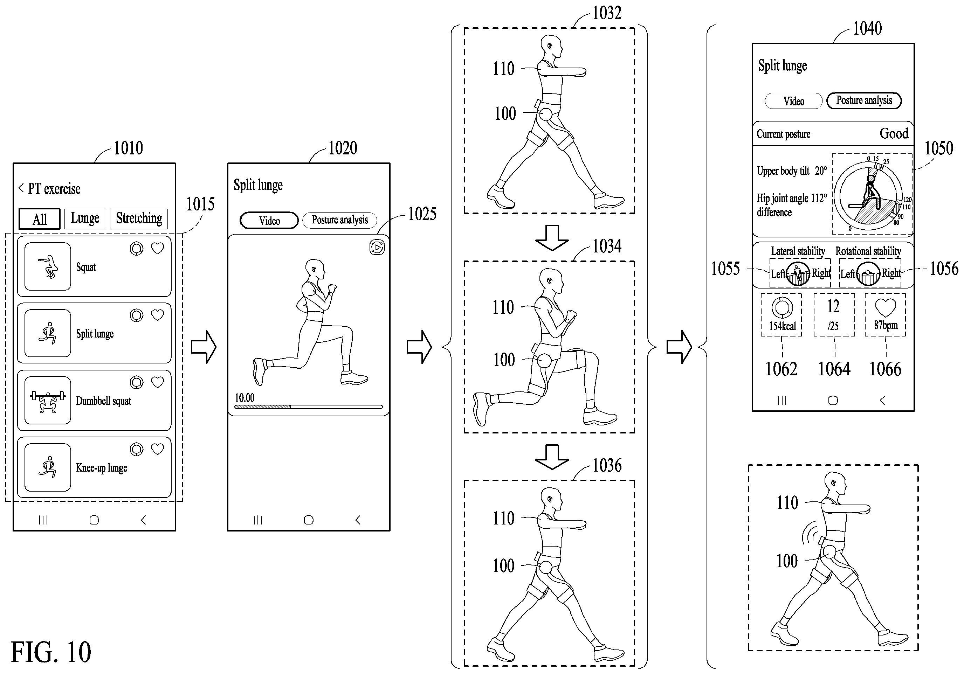

FIG. 10 is a diagram illustrating an example of providing exercise posture evaluation information of a user through a GUI of an electronic device and through a wearable device according to an example embodiment.

Referring to FIG. 10, the electronic device 210 may assist the user 110 in performing an exercise through an application and provide a GUI for evaluating an exercise posture of the user 110. Through one screen 1010 of the GUI, the electronic device 210 may provide a list 1015 of exercise programs from which the user 110 may select.

When the user 110 selects an exercise program to be performed, the electronic device 210 may provide a guide image 1025 for guiding a process of exercise postures of an exercise program selected by the user 110 through one screen 1020 of the GUI. The guide image 1025 may be provided at the request of the user 110 before the user 110 starts performing an exercise posture, while the user 110 is performing the exercise posture, or after the user 110 ends performing the exercise posture.

The user 110 may perform a series of exercise postures according to the selected exercise program. For example, when the user 110 selects an exercise program of split lunge, the user 110 may perform a series of exercise postures with the wearable device 100 worn a body of the user 110, starting with a basic posture 1032 of standing with a right leg and a left leg spread forward and backward, through an intermediate posture 1034 of bending the left leg while stepping it forward, and ending with a basic posture 1036 of standing again with the right leg and the left leg spread forward and backward. The series of exercise postures may correspond to one time of performing the exercise postures defined for the split lunge. The user 110 may repeatedly perform the series of exercise postures until they reach a target number of repetitions.

When the user 110 performs the series of exercise postures, the wearable device 100 may measure an upper body tilt value and a hip joint angle value of each of both hip joints of the user 110 through an IMU and an angle sensor. The measured upper body tilt value and hip joint angle value may be transmitted to the electronic device 210.

The electronic device 210 may analyze sensor data received from the wearable device 100 to evaluate an exercise posture (e.g., the intermediate posture 1034) performed by the user 110, and may then display a result of the evaluation through at least one visual guide object. One screen 1040 of a GUI including the at least one visual guide object is illustrated. The screen 1040 may display a first visual guide object 1050 indicating an exercise posture reference for the split lunge selected by the user 110 and an exercise posture measurement value of the user 110. The screen 1040 may also display second visual guide objects 1055 and 1056 indicating physical movement stability of the user 110 evaluated based on the sensor data of the wearable device 100. The second visual guide object 1055 may indicate lateral (or left-right) stability of a physical movement of the user 110, and the second visual guide object 1056 may indicate rotational stability of the physical movement of the user 110.

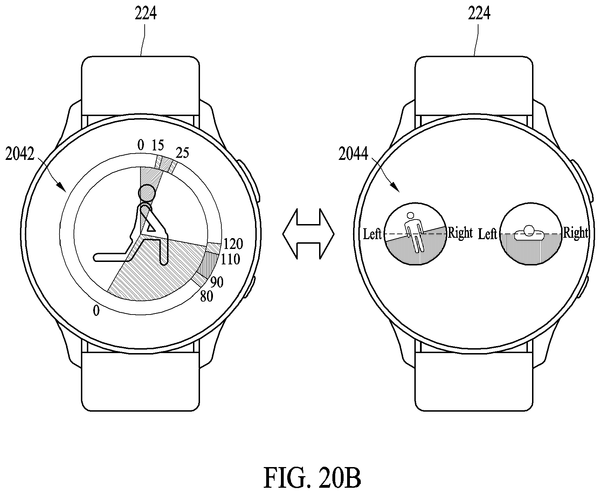

The screen 1040 of the GUI may also display an exercise posture evaluation level (e.g., great, good, or bad) of the user 110 and an exercise posture measurement value (e.g., an upper body tilt value and a hip joint angle difference value between both hip joints) determined based on the sensor data. The screen 1040 may also display at least one of a calorie consumption estimate 1062 indicating calories consumed by the exercise performed up to a current point in time, the number 1064 of repetitions of an exercise posture performed up to the current point in time, or a heart rate 1066. The heart rate 1066 may be determined through an analysis of a biosignal obtained from a sensor of a smartwatch (e.g., a smartwatch 224 of FIG. 20B) or a smart band worn on the user 110.

When an immediately previous exercise posture is completed, the electronic device 210 may transmit exercise posture evaluation information associated with the immediately previous exercise posture to the wearable device 100. The wearable device 100 may then provide the user 110 with a feedback signal corresponding to the received exercise posture evaluation information. For example, the wearable device 100 may reproduce a notification sound corresponding to "great," "good," or "bad" according to the exercise posture evaluation level of the user 110.

The user 110 may do an exercise freely without restrictions of location or space through the wearable device 100 and the electronic device 210 and clearly recognize a body part for which an exercise posture needs to be corrected and recognize a target exercise posture reference, and may thereby correct an exercise posture they take in real time. The wearable device 100 may be used to accurately measure an exercise posture of the user 110, provide feedback information on the evaluation of the exercise posture to the user 110 through graphic elements for each exercise posture element, and enable professional coaching for the exercise posture.

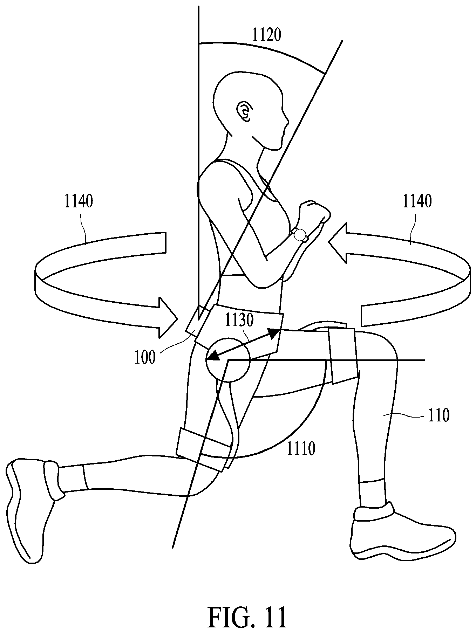

FIG. 11 is a diagram illustrating an example of evaluating an exercise posture of a user according to an example embodiment.

Referring to FIG. 11, when the user 110 performs an exercise program of split lunge with the wearable device 100 worn on a body of the user 110, an intermediate posture of bending a left leg while stepping it forward is illustrated. In an example embodiment, the wearable device 100 may obtain sensor data including physical movement information of the user 110 from an exercise posture such as the intermediate posture of the split lunge through sensors, and transmit the obtained sensor data to the electronic device 210. The electronic device 210 may evaluate the exercise posture of the user based on the sensor data obtained through the wearable device 100.

In an example embodiment, the electronic device 210 may evaluate an exercise posture of the user 110 with respect to various evaluation elements for exercise postures of the split lunge. For example, the electronic device 210 may evaluate a hip joint angle difference 1110 between both hip joints of the user 110 based on the sensor data. A hip joint angle difference described herein may be an evaluation element used for determining whether a leg of a user gets down and gets up at an appropriate angle when the user is performing an exercise posture. The electronic device 210 may evaluate an upper body tilt 1120 of the user 110 based on the sensor data. An upper body tilt described herein may be an evaluation element used for determining whether a pelvis of a user tilts forward/backward when the user is performing an exercise posture. The electronic device 210 may evaluate lateral stability 1130 of a physical movement of the user 110 based on the sensor data. Lateral stability described herein may be an evaluation element used for determining whether a pelvis of a user tilts leftward/rightward when the user is performing an exercise posture. The electronic device 210 may evaluate rotational stability 1140 of a physical movement of the user 110 based on the sensor data. Rotational stability described herein may be an evaluation element used for determining whether a pelvis of a user is rotated when the user is performing an exercise posture.

In an example embodiment, the electronic device 210 may evaluate an exercise posture of the user 110 according to an evaluation reference shown in Table 1 below for each of the evaluation elements described above. The electronic device 210 may determine an evaluation level to which an exercise posture measurement value of the user 110 belongs, for each evaluation element, according to a reference of each evaluation element. The evaluation reference shown below is provided merely as an example and is not limited thereto.

**[Table 1]**

| Reference and evaluation level | Hip joint angle difference | Upper body tilt | Lateral stability | Rotational stability |
|---|---|---|---|---|
| Reference | 90∼110° | Pelvic obliquity angle-20° | Parallel | Parallel |
| Great | 90∼110° | Referemce±3 ° | Referemce± 10° | Referemce± 10° |
| Good | 80∼89°, 111∼120° | Referemce±5° | Referemce± 20° | Referemce± 20° |
| Bad | Other ranges | Other ranges | Other ranges | Other ranges |

FIG. 12 is a diagram illustrating an example of a screen of a GUI including visual guide objects according to an example embodiment.

Referring to FIG. 12, a screen 1200 of a GUI may provide a first visual guide object 1210 indicating an exercise posture reference for an exercise program performed by a user and an exercise posture measurement value of the user. The screen 1200 may further provide a second visual guide object 1220 indicating lateral stability of a physical movement measured from an exercise posture of the user and a second visual guide object 1230 indicating rotational stability of the physical movement. The screen 1200 may also provide a comprehensive evaluation level 1240 (e.g., great, good, or bad) of the exercise posture of the user and an exercise posture measurement value 1215 measured from the exercise posture of the user. For example, the exercise posture measurement value 1215 displayed on the screen 1200 may include a maximum upper body tilt value of the user measured through an IMU of the wearable device 100 in a process in which the user is performing an immediately previous exercise posture and a maximum hip joint angle difference value between both hip joints of the user measured through an angle sensor of the wearable device 100 in the process in which the user is performing the immediately previous exercise posture. In an example embodiment, indicators displayed in the first visual guide object 1210, the evaluation level 1240, and colors of the second visual guide objects 1220 and 1230 may change according to a result of evaluating each evaluation element for an exercise posture of the user.

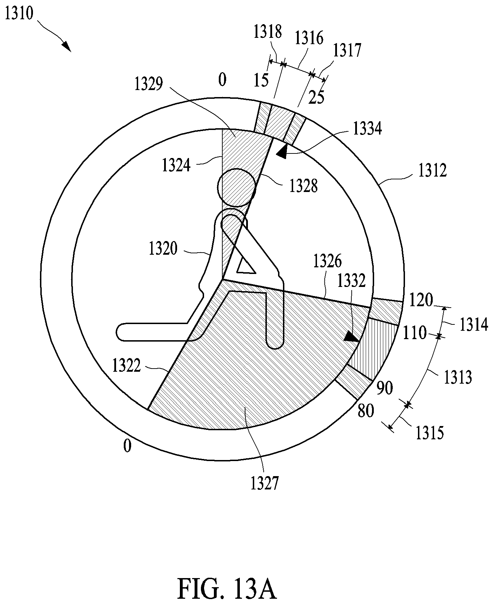

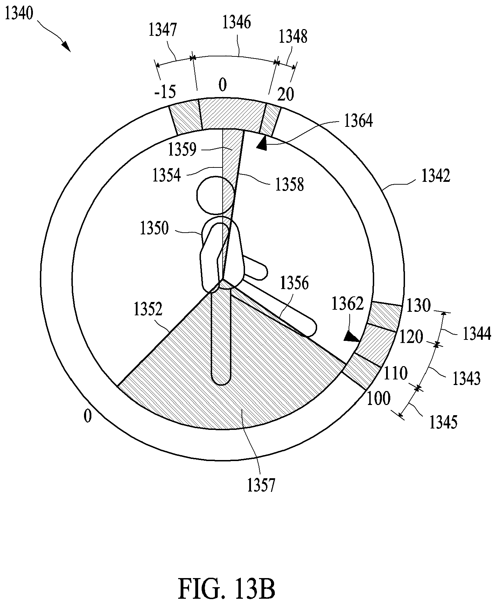

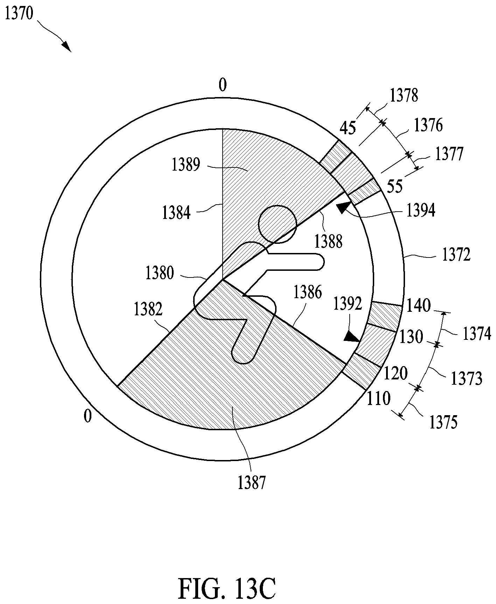

FIGS. 13A, 13B, and 13C are diagrams illustrating various examples of a first visual guide object according to an example embodiment.

A first visual guide object 1310 shown in FIG. 13A, a first visual guide object 1340 shown in FIG. 13B, and a first visual guide object 1370 shown in FIG. 13C are provided as various examples of a first visual guide object that is displayed for each type of exercise posture of a user.

The first visual guide object 1310 shown in FIG. 13A may be an example first visual guide object that is displayed when the user performs an exercise program (e.g., split lunge, reverse lunge, or split jack) by which the user moves both legs separately. The first visual guide object 1310 is provided as an example of a first visual guide object displayed when the user performs split lunge.

In an example embodiment, the first visual guide object 1310 may indicate an exercise posture reference for the exercise program (e.g., split lunge) and an exercise posture measurement value of the user, based on a circular edge area 1312. However, the shape of the edge area 1312 is not limited to a circle but may vary. In a central portion of the first visual guide object 1310, an avatar object (or an icon object) 1320 showing a representative posture of the exercise program (e.g., split lunge) performed by the user may be displayed, and a posture of the avatar object 1320 may be displayed differently according to a type of exercise program.

In an example embodiment, the first visual guide object 1310 may include first indicators 1313, 1314, 1315, 1316, 1317, and 1318 indicating exercise posture references for the exercise program selected by the user. The first indicators 1313, 1314, 1315, 1316, 1317, and 1318 may be displayed near the edge area 1312. The first indicators 1313, 1314, 1315, 1316, 1317, and 1318 may indicate a plurality of exercise posture reference ranges corresponding to different exercise posture evaluation levels. For example, the first indicators 1314 and 1315 may indicate an exercise posture reference range corresponding to an evaluation level of "good" for a hip joint angle difference value, and the first indicator 1313 may indicate an exercise posture reference range corresponding to an evaluation level of "great" for the hip joint angle difference value. In addition, the first indicators 1317 and 1318 may indicate an exercise posture reference range corresponding to an evaluation level of "good" for an upper body tilt value, and the first indicator 1316 may indicate an exercise posture reference range corresponding to an evaluation level of "great" for the upper body tilt value. When the user selects a specific exercise program, the electronic device 210 may display, on the first visual guide object 1310, a plurality of exercise posture reference ranges corresponding to different exercise posture evaluation levels based on exercise posture references for the selected exercise program (e.g., split lunge). In an example embodiment, the first indicators 1313, 1314, 1315, 1316, 1317, and 1318 may be displayed in different colors according to the evaluation levels respectively corresponding to the first indicators 1313, 1314, 1315, 1316, 1317, and 1318. Around the first indicators 1313, 1314, 1315, 1316, 1317, and 1318, reference values 80, 90, 110, 120, 15, and 25 that define the exercise posture reference ranges may be displayed.

In an example embodiment, the first visual guide object 1310 may include second indicators 1326, 1328, 1332, and 1334 indicating exercise posture measurement values of the user. For example, the second indicator 1326 may indicate a maximum hip joint angle difference value between both hip joints of the user determined from a previous exercise posture, and the second indicator 1328 may indicate a maximum upper body tilt value of the user determined from the previous exercise posture. The second indicator 1332 may indicate in real time a current hip joint angle difference value between both hip joints determined from a current exercise posture of the user, and the second indicator 1334 may indicate in real time a current upper body tilt value determined from the current exercise posture of the user. In an example embodiment, each of the second indicators 1326, 1328, 1332, and 1334 may have a color to be displayed differently according to an evaluation reference range to which a corresponding exercise posture measurement value belongs.

In an example embodiment, the first visual guide object 1310 may include third indicators 1327 and 1329. The third indicator 1327 may indicate an angle range that is defined by a reference line 1322 for a hip joint angle difference value between both hip joints of the user and an angle line (e.g., the second indicator 1326) corresponding to the maximum hip joint angle difference value between both hip joints of the user determined from the previous exercise posture of the user. The third indicator 1329 may indicate an angle range that is defined by a reference line 1324 (e.g., a vertical line) for an upper body tilt value of the user and an angle line (e.g., the second indicator 1328) corresponding to the maximum upper body tilt value of the user determined from the previous exercise posture of the user. Each of the third indicators 1327 and 1329 may have a color to be displayed differently according to a corresponding evaluation reference range to which each of the second indicators 1326 and 1328 belongs. For example, the color of the third indicator 1329 may be displayed in blue when a result of evaluating an upper body posture of the user from the previous exercise posture is "great," in green when the result of the evaluation is "good," and in yellow when the result of the evaluation is "bad." By the colors of the third indicators 1327 and 1329, the user may intuitively recognize respective evaluation levels of an upper body posture and a leg posture in the previous exercise posture of the user. In an example embodiment, the third indicators 1327 and 1329 may be displayed in a fan shape, but the shape of which is not limited thereto.

The first visual guide object 1340 shown in FIG. 13B may be an example first visual guide object that is displayed when the user performs an exercise program (e.g., leg swing, knee-up lunge, single leg sprint, or straight leg kick back) by which the user moves only one of both legs of the user. The first visual guide object 1340 is provided as an example of a first visual guide object displayed when the user performs leg swing.

In an example embodiment, the first visual guide object 1340 may indicate an exercise posture reference for the exercise program (e.g., leg swing) and an exercise posture measurement value of the user, based on a circular edge area 1342. In a central portion of the first visual guide object 1340, an avatar object 1350 showing a representative posture of the exercise program (e.g., leg swing) performed by the user may be displayed.

In an example embodiment, the first visual guide object 1340 may include first indicators 1343, 1344, and 1345 indicating exercise posture reference ranges for a hip joint angle difference between both hip joints in the exercise program selected by the user, and first indicators 1346, 1347, and 1348 indicating exercise posture reference ranges for an upper body tilt in the exercise program. In an example embodiment, each of the first indicators 1343, 1344, 1345, 1346, 1347, and 1348 may be displayed in different colors according to a corresponding evaluation level.

In an example embodiment, the first visual guide object 1340 may include a second indicator 1356 indicating a maximum hip joint angle difference value between both hip joints of the user determined from a previous exercise posture and a second indicator 1358 indicating a maximum upper body tilt value of the user determined from the previous exercise posture. In an example embodiment, the first visual guide object 1340 may include a second indicator 1362 indicating in real time a current hip joint angle difference value between both hip joints determined from a current exercise posture of the user and a second indicator 1364 indicating in real time a current upper body tilt value determined from the current exercise posture of the user. In an example embodiment, each of the second indicators 1356, 1358, 1362, and 1364 may be displayed in different colors according to an evaluation reference range to which a corresponding exercise posture measurement value belongs.

Each embodiment herein may be used in combination with any other embodiment(s) described herein.

In an example embodiment, the first visual guide object 1340 may include a third indicator 1357 indicating an angle range that is defined by a reference line 1352 for a hip joint angle difference value between both hip joints of the user and an angle line (e.g., the second indicator 1356) corresponding to the maximum hip joint angle difference value between both hip joints of the user determined from the previous exercise posture of the user. The first visual guide object 1340 may include a third indicator 1359 indicating an angle range that is defined by a reference line 1354 for an upper body tilt value of the user and an angle line (e.g., the second indicator 1358) corresponding to the maximum upper body tilt value of the user determined from the previous exercise posture of the user. Each of the third indicators 1357 and 1359 may be displayed in different colors according to an evaluation reference range to which the second indicators 1356 and 1358 belong, respectively.

The first visual guide object 1370 shown in FIG. 13C may be an example first visual guide object that is displayed when the user performs an exercise program (e.g., squat, half squat, or kneeling squat) by which the user moves both legs simultaneously. The first visual guide object 1370 is provided as an example of a first visual guide object displayed when the user performs half squat.

In an example embodiment, the first visual guide object 1370 may indicate an exercise posture reference for the exercise program (e.g., half squat) and an exercise posture measurement value of the user, based on a circular edge area 1372. In a central position of the first visual guide object 1370, an avatar object 1380 showing a representative posture in the exercise program (e.g., half squat) performed by the user may be displayed.

In an example embodiment, the first visual guide object 1370 may include first indicators 1373, 1374, and 1375 indicating exercise posture reference ranges for a hip joint angle difference in the exercise program selected by the user and first indicators 1376, 1377, and 1378 indicating exercise posture reference ranges for an upper body tilt of the user. In an example embodiment, the first indicators 1373, 1374, 1375, 1376, 1377, and 1378 may be displayed in different colors according to a corresponding evaluation level.

In an example embodiment, the first visual guide object 1370 may include a second indicator 1386 indicating a maximum hip joint angle difference value between both hip joints of the user determined from a previous exercise posture of the user and a second indicator 1388 indicating a maximum upper body tilt value determined from the previous exercise posture. In an example embodiment, the first visual guide object 1370 may include a second indicator 1392 indicating in real time a current hip joint angle difference value between both hip joints determined from a current exercise posture of the user and a second indicator 1394 indicating in real time a current upper body tilt value determined from the current exercise posture. In an example embodiment, each of the second indicators 1386, 1388, 1392, and 1394 may be displayed in different colors according to an evaluation reference range to which a corresponding exercise posture measurement value belongs.

In an example embodiment, the first visual guide object 1370 may include a third indicator 1387 indicating an angle range that is defined by a reference line 1382 for a hip joint angle difference value between both hip joints of the user and an angle line (e.g., the second indicator 1386) corresponding to the maximum hip joint angle difference value between both hip joints of the user determined from the previous exercise posture of the user. The first visual guide object 1370 may also include a third indicator 1389 indicating an angle range that is defined by a reference line 1384 for an upper body tilt value of the user and an angle line (e.g., the second indicator 1388) corresponding to the maximum upper body tilt value of the user determined from the previous exercise posture of the user. Each of the third indicators 1387 and 1389 may be displayed in different colors according to an evaluation reference range to which the second indicators 1386 and 1388 belong, respectively.

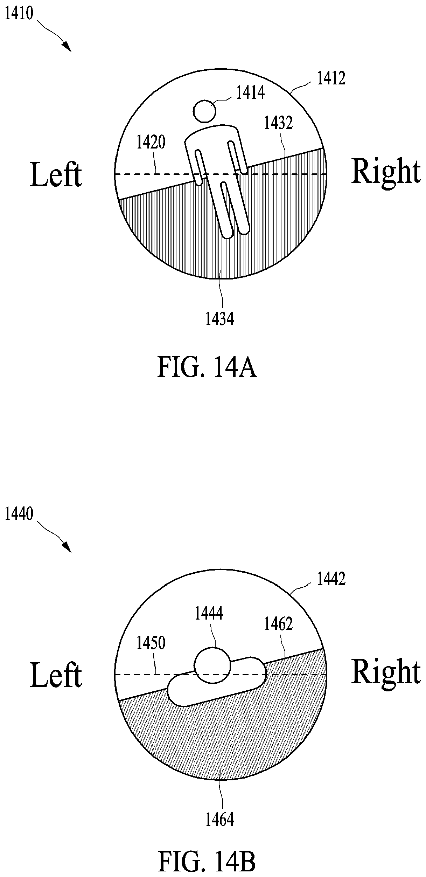

FIGS. 14A and 14B are diagrams illustrating examples of a second visual guide object according to an example embodiment.

FIG. 14A shows an example second visual guide object 1410 indicating lateral (or left-right) stability of a physical movement of a user. In an example embodiment, the second visual guide object 1410 may be displayed as a circle that is defined by an edge area 1412 but is not limited thereto. The second visual guide object 1410 may include a fixed reference line 1420 (e.g., a horizontal line), an avatar object 1414 of which a tilt to be displayed changes according to an exercise posture measurement value corresponding to the lateral stability of the physical movement, and a color object 1434 of which a color changes according to an exercise posture evaluation level of the exercise posture measurement value corresponding to the lateral stability of the physical movement. For example, the color object 1434 may be displayed in blue when the exercise posture evaluation level of the lateral stability of the physical movement is "great," in green when the exercise posture evaluation level of the lateral stability is "good," and in yellow when the exercise posture evaluation level of the lateral stability is "bad." The avatar object 1414 and the color object 1434 may each have a tilt to be displayed differently according to a degree of the lateral stability of the physical movement of the user measured based on sensor data. For example, the avatar object 1414 may be displayed as an icon corresponding to a frontal shape of a person. An area of the color object 1434 may be defined by a line segment 1432 of which a tilt changes according to the degree of the lateral stability and by the edge area 1412. In an example embodiment, the color object 1434 may have a semicircular shape but is not limited thereto.

FIG. 14B shows an example of a second visual guide object 1440 indicating rotational stability of a physical movement of a user. In an example embodiment, the second visual guide object 1440 may be displayed as a circle that is defined by an edge area 1442 but is not limited thereto. The second visual guide object 1440 may include a fixed reference line 1450 (e.g., a horizontal line), an avatar object 1444 of which a tilt to be displayed changes according to an exercise posture measurement value corresponding to the rotational stability of the physical movement, and a color object 1464 of which a color changes according to an exercise posture evaluation level of the exercise posture measurement value corresponding to the rotational stability of the physical movement. The avatar object 1444 and the color object 1464 may each have a tilt to be displayed differently according to a degree of the rotational stability of the physical movement of the user measured based on sensor data. For example, the avatar object 1444 may be displayed as an icon corresponding to a shape of a person viewed from above. An area of the color object 1464 may be defined by a line segment 1462 of which a tilt changes according to the degree of the rotational stability and by the edge area 1442. In an example embodiment, the color object 1464 may have a semicircular shape but is not limited thereto.

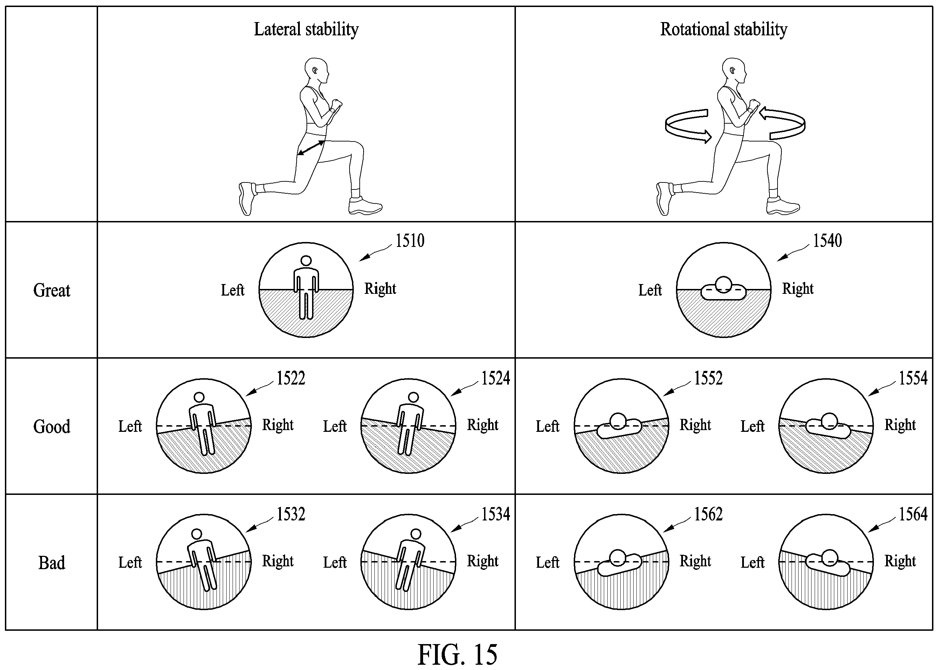

FIG. 15 is a diagram illustrating various examples of a second visual guide object based on a physical movement stability evaluation for a user according to an example embodiment.

FIG. 15 shows various examples of displaying a second visual guide object according to an evaluation level of lateral stability and an evaluation level of rotational stability of a physical movement of a user by the electronic device 210 when the user performs exercise postures of split lunge. In an example embodiment, the electronic device 210 may recognize a lateral tilt and a rotation angle of the physical movement of the user through an IMU of the wearable device 100, and may provide, through a GUI, a second visual guide object indicating each of the lateral stability and the rotational stability based on the lateral tilt and the rotation angle recognized by the wearable device 100. The electronic device 210 may output different representations of the second visual guide object according to the evaluation level corresponding to the lateral stability or the rotational stability.

In an example embodiment, when the evaluation level corresponding to the lateral stability is "great," the second visual guide object may be displayed as a second visual guide object 1510. When the evaluation level corresponding to the lateral stability is "good," the second visual guide object may be displayed as a second visual guide object 1522 or a second visual guide object 1524 according to a direction in which the body of the user is tilted (e.g., to the right or left). When the evaluation level corresponding to the lateral stability is "bad," the second visual guide object may be displayed as a second visual guide object 1532 or a second visual guide object 1534 according to a direction in which the body of the user is tilted. An avatar object and a color object may be displayed as being more tilted in the second visual guide object 1532 than in the second visual guide object 1522, and may be displayed as being more tilted in the second visual guide object 1534 than in the second visual guide object 1524. For example, the color object of the second visual guide object 1510 may be displayed in blue, the color objects of the second visual guide objects 1522 and 1524 may be displayed in green, and the color objects of the second visual guide objects 1532 and 1534 may be displayed in yellow, but examples are not limited thereto.

In an example embodiment, when the evaluation level corresponding to the rotational stability is "great," the second visual guide object may be displayed as a second visual guide object 1540. When the evaluation level corresponding to the rotational stability is "good," the second visual guide object may be displayed as a second visual guide object 1552 or a second visual guide object 1554 according to a direction in which the body of the user is rotated (e.g., clockwise or counterclockwise). When the evaluation level corresponding to the rotational stability is "bad," the second visual guide object may be displayed as a second visual guide object 1562 or a second visual guide object 1564 according to a direction in which the body is rotated. An avatar object and a color object may be displayed as being more tilted in the second visual guide object 1562 than in the second visual guide object 1552, and may be displayed as being more tilted in the second visual guide object 1564 than in the second visual guide object 1554. For example, the color object of the second visual guide object 1540 may be displayed in blue, the color objects of the second visual guide objects 1552 and 1554 may be displayed in green, and the color objects of the second visual guide objects 1562 and 1564 may be displayed in yellow, but examples are not limited thereto.

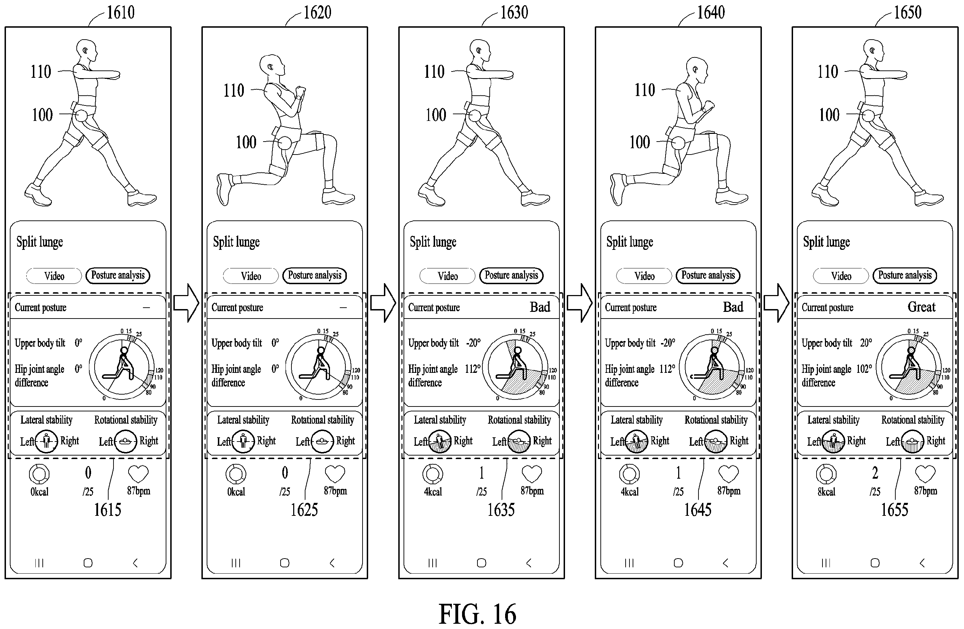

FIG. 16 is a diagram illustrating an example of a change in visual guide objects by an exercise performed by a user according to an example embodiment.

FIG. 16 shows a change in a visual guide object according to an exercise posture of the user 110 when the user 110 is performing exercise postures of split lunge with the wearable device 100 worn on the body of the user 110. In a state 1610 in which the user 110 is preparing for a first round of exercise postures of the split lunge, a first visual guide object indicating exercise posture reference ranges corresponding to exercise posture references of the split lunge may be output on an area 1615 of a GUI provided through the electronic device 210. Since the exercise postures have not been performed once yet, an exercise posture measurement value of the user 110 may not be displayed on the first visual guide object and a second visual guide object in the area 1615.

In a state 1620 in which the user 110 is performing the exercise postures after starting the first round of the exercise postures of the split lunge, physical movement information of the user 110 may be measured through sensors (e.g., an IMU and an angle sensor) of the wearable device 100, and sensor data including the physical movement information may be transmitted to the electronic device 210. The user 110 has not yet completed the first round of the exercise postures by that point in time, and thus one area 1625 providing exercise posture evaluation information of the user 110 may remain the same as the area 1615 in the previous state 1610.

In a state 1630 in which the user 110 is preparing for a second round of exercise postures after completing the first round of the exercise postures, the electronic device 210 may analyze the sensor data of the wearable device 100 obtained during the first round of the exercise postures of the user 110 and evaluate the first round of the exercise postures of the user 110. The electronic device 210 may provide exercise posture evaluation information obtained by evaluating the exercise postures of the user 110 through one area 1635 of the GUI. A screen (e.g., the area 1635) may provide, through second indicators, exercise posture measurement values (e.g., an upper body tilt value, a hip joint angle difference value between both hip joints, lateral stability of a physical movement, and rotational stability of the physical movement) for the first round of the exercise postures of the user 110. In this case, when it is determined that a comprehensive evaluation level for the first round of the exercise postures is "bad," an evaluation level of "bad" may be displayed on the area 1635. When the first round of the exercise postures has been completed, the number of repetitions of the exercise postures may be updated to be "1," and a calorie consumption estimate indicating calories that have been consumed up to the first round of the exercise postures may be displayed through the GUI.

In a state 1640 in which the user 110 is performing the exercise postures of the split lunge after starting the second round of the exercise postures, physical movement information of the user 110 may be measured again through the sensors of the wearable device 100, and sensor data including the physical movement information may be transmitted to the electronic device 210. The second round of the exercise postures of the user 110 has not yet been completed by that point in time, and thus one area 1645 providing exercise posture evaluation information may remain the same as the area 1635 in the previous state 1640.

In a state 1650 in which the user 110 is preparing for a third round of exercise postures after completing the second round of the exercise postures, the electronic device 210 may analyze the sensor data of the wearable device 100 obtained during the second round of the exercise postures of the user 110 and evaluate the second round of the exercise postures of the user 110. The electronic device 210 may provide exercise posture evaluation information obtained by evaluating the exercise postures of the user 110 to the user 110 through one area 1655 of the GUI. A screen (e.g., the area 1655) may provide, through second indicators, exercise posture measurement values for the second round of the exercise postures of the user 110. When it is determined that a comprehensive evaluation level for the second round of the exercise postures is "great," the evaluation level of "great" may be displayed on the area 1655. When the second round of the exercise postures has been completed, the number of repetitions of the exercise postures may be updated to be "2," and a calorie consumption estimate indicating calories that have been consumed up to the second round of the exercise postures may be displayed through the GUI.

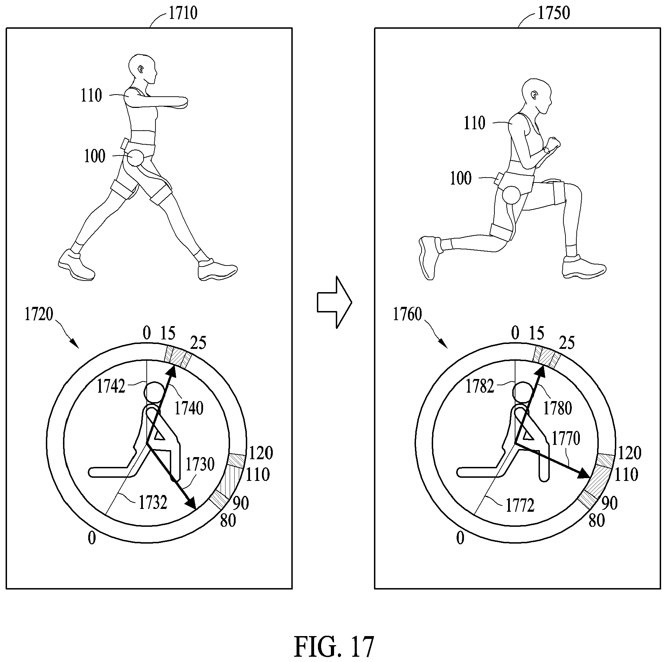

FIG. 17 is a diagram illustrating an example of a first visual guide object providing exercise posture evaluation information of a user in the form of a gauge according to an example embodiment.

FIG. 17 shows a change in a first visual guide object according to an exercise posture of the user 110 when the user 110 is performing exercise postures of split lunge with the wearable device 100 worn on a body of the user 110. In an example embodiment, the first visual guide object may display an exercise posture measurement value of a current exercise posture of the user 110. A second indicator indicating the exercise posture measurement value may be provided in the form of a gauge but is not limited thereto.

In a state 1710 in which the user 110 is performing exercise postures of the split lunge, a first visual guide object 1720 may display a second indicator 1730 that indicates a hip joint angle difference value between both hip joints of the user 110 measured from a current exercise posture of the user 110 and a second indicator 1740 that indicates an upper body tilt value of the user 110 measured from the current exercise posture, along with exercise posture reference ranges corresponding to exercise posture references for the split lunge. As the exercise posture of the user 110 changes, exercise posture measurement values indicated by the second indicator 1730 and the second indicator 1740 in real time may change. The second indicators 1730 and 1740 may indicate in real time exercise posture measurement values of the user 110. In an example embodiment, the first visual guide object 1720 may include a third indicator that indicates an angle range defined by a reference line 1732 and the second indicator 1730 and a third indicator that indicates an angle range defined by a reference line 1742 and the second indicator 1740.

In a state 1750 in which the user 110 is performing an intermediate posture of bending a left leg of the user 110 while stepping it forward among exercise postures of the split lunge, a first visual guide object 1760 may display a second indicator 1770 that indicates a hip joint angle difference value between both hip joints of the user 110 measured from the current intermediate posture and a second indicator 1780 that indicates an upper body tilt value of the user 110 measured from the current intermediate posture, along with exercise posture reference ranges corresponding to exercise posture references of the split lunge.

In an example embodiment, when the hip joint angle difference value indicated by the second indicator 1770 is included in an exercise posture reference range indicated by a first indicator, the second indicator 1770 may change to a color corresponding to the exercise posture reference range. When the upper body tilt value indicated by the second indicator 1780 is included in an exercise posture reference range indicated by the first indicator, the second indicator 1780 may change to a color corresponding to the exercise posture reference range. Since the second indicators 1770 and 1780 reflect in real time an exercise posture of the user 110, the user 110 may immediately attempt to correct the exercise posture based on the change in the second indicators 1770 and 1780 in the middle of performing the exercise posture, and effectively recognize in real time a degree by which the correction is required. In addition, when the color of the second indicators 1770 ad 1780 change as the second indicators 1770 and 1780 are included in an exercise posture reference range corresponding to an evaluation level of "good" or "great" while the user 110 is performing the intermediate posture, the user 110 may effectively recognize that it is time to return to a basic posture after completing the intermediate posture.

In an example embodiment, the first visual guide object 1760 may include a third indicator that indicates an angle range defined by the reference line 1772 and the second indicator 1770 and a third indicator that indicates an angle range defined by the reference line 1782 and the second indicator 1780. The reference line 1772 may correspond to the reference line 1732, and the reference line 1782 may correspond to the reference line 1742.

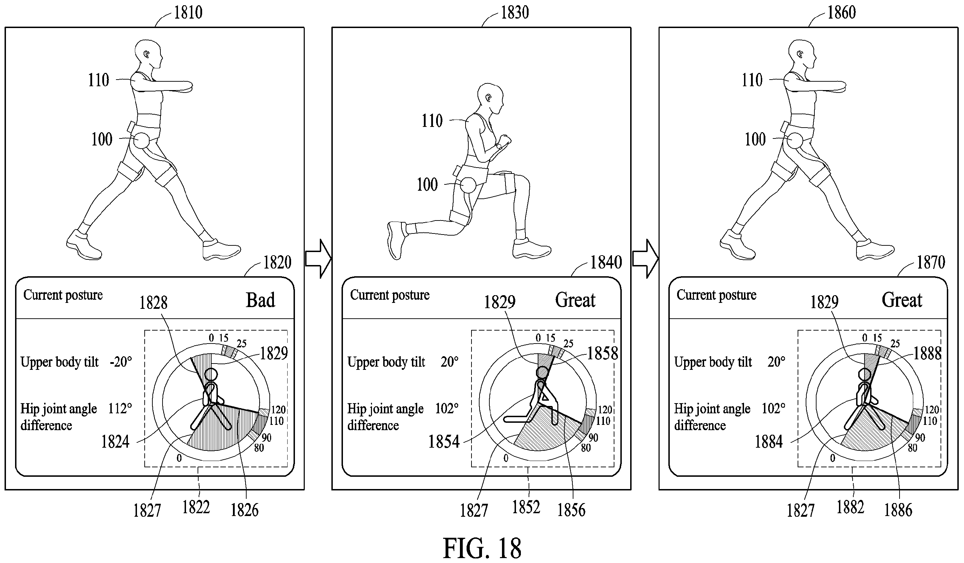

FIG. 18 is a diagram illustrating an example of a change in an avatar object in a first visual guide object based on an exercise posture of a user according to an example embodiment.

FIG. 18 shows a change in a visual guide object according to an exercise posture of the user 110 when the user 110 performs exercise postures of split lunge with the wearable device 100 worn on a body of the user 110. In a state 1810 in which, after completing a first round of exercise postures of the split lunge, the user 110 is preparing for a second round of exercise postures of the split lunge, the electronic device 210 ma provide exercise posture evaluation information determined while the user 110 is performing the first round of exercise postures through one area 1820 of a GUI.

On the area 1820 of the GUI, a first visual guide object 1822 for visually displaying the exercise posture evaluation information of the user 110 may be displayed. An avatar object 1824 included in the first visual guide object 1822 may reflect therein a current posture of the user 110 in real time. The shape of the avatar object 1824 may change in shape to correspond to the current posture (e.g., a leg posture in particular) of the user 110. The change in the shape of the avatar object 1824 may allow the user 110 to intuitively recognize their current posture. The first visual guide object 1822 may display a second indicator 1826 indicating a maximum hip joint angle difference value evaluated from the previous first round of exercise postures of the user 110, a second indicator 1828 indicating a maximum upper body tilt value evaluated from the previous first round of exercise postures, and reference lines 1827 and 1829. The first visual guide object 1822 may display third indicators defined by the reference lines 1827 and 1829 and the second indicators 1826 and 1828.

In a state 1830 in which, after starting the second round of exercise postures, the user 110 is performing an intermediate posture of bending a left leg while stepping it forward among the exercise postures of the split lunge, physical movement information of the user 110 may be measured again through sensors of the wearable device 100, and sensor data including the physical movement information may be transmitted to the electronic device 210. In this case, a first visual guide object 1852 displayed on one area 1840 of the GUI may display in real time exercise movement evaluation information for the second round of exercise postures of the user 110. An avatar object 1854 included in the first visual guide object 1852 may change in shape according to a current posture of the user 110. In the state 1830, a posture of the avatar object 1854 may also change to correspond to a shape corresponding to the intermediate posture of bending the left leg while stepping it forward that is a posture of the user 110.

The first visual guide object 1852 may display a second indicator 1856 that indicates a hip joint angle difference value evaluated from the current second round of exercise postures of the user 110, a second indicator 1858 that indicates an upper body tilt value evaluated from the current second round of exercise postures, and reference lines 1827 and 1829. The second indicators 1856 and 1858 may change in real time according to an exercise posture of the user 110. The first visual guide object 1852 may display third indicators defined by the reference lines 1827 and 1829 and the second indicators 1856 and 1858. In an example embodiment, during the second round of exercise postures, exercise posture evaluation information associated with a hip joint angle difference value may be determined at the time when the hip joint angle difference value reaches the maximum hip joint angle difference value, and exercise posture evaluation information associated with an upper body tilt value may be determined at the time when the upper body tilt value reaches the maximum upper body tilt value.

In a state 1860 in which, after completing the second round of exercise postures of the split lunge, the user 110 is preparing for a third round of exercise postures of the split lunge, the electronic device 210 may provide exercise posture evaluation information determined while the user 110 is performing the second round of exercise postures through one area 1870 of the GUI. A first visual guide object 1882 provided on the area 1870 may display a second indicator 1886 that indicates a maximum hip joint angle difference value determined during the second round of exercise postures of the user 110 and a second indicator 1888 that indicates a maximum upper body tilt value determined during the second round of exercise postures of the user 110. An avatar object 1884 may change in shape to correspond to a current posture of the user 110. The first visual guide object 1882 may display third indicators defined by the reference lines 1827 and 1829 and the second indicators 1886 and 1888.

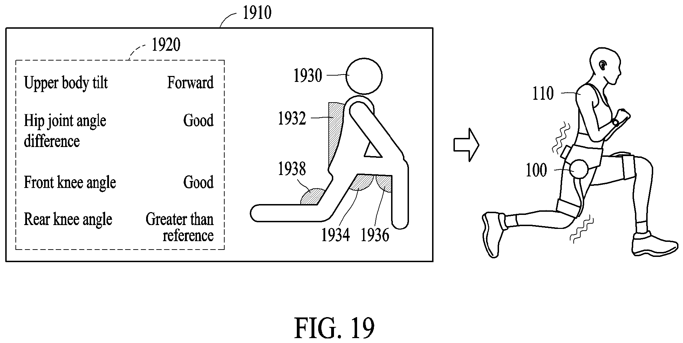

FIG. 19 is a diagram illustrating an example of providing exercise posture evaluation information of a user through an avatar object and a wearable device according to an example embodiment.

Referring to FIG. 19, on one screen 1910 of a GUI provided by the electronic device 210, exercise posture evaluation information 1920 determined for each exercise posture evaluation reference for the user 110 may be provided through an avatar object 1930. In an example embodiment, the avatar object 1930 may display in real time evaluation information associated with an exercise posture of the user 110. Exercise posture evaluation information for each body part of the user 110 may be provided by each body part of the avatar object 1930 through graphic elements 1932, 1934, 1936, and 1938. Each of the graphic elements 1932, 1934, 1936, and 1938 may change in color according to an evaluation level determined according to the exercise posture evaluation reference for each body part. The graphic elements 1932, 1934, 1936, and 1938 may be displayed in a fan shape for each body part, and may intuitively display a body part requiring exercise posture correction for each body part in an evaluation of exercise postures of the user 110.

According to an example embodiment, information about a body part requiring exercise posture correction may be transmitted from the electronic device 210 to the wearable device 100, and the wearable device 100 may generate haptic feedback (e.g., vibration) at the body part requiring exercise posture correction through a haptic module. For example, when it is determined that an upper body tilt and a knee angle of a right leg need to be corrected as a result of evaluating an exercise posture of the user 110, the wearable device 100 may generate haptic feedback around a waist of the user 110 and around a first thigh fastener. The wearable device 100 may also reproduce a guide voice (e.g., "please raise your upper body more" or "please lower your right leg more") for guiding the user 110 to how to correct the exercise posture through a sound output module. The user 110 may thus immediately recognize the body part requiring the correction during the exercise posture through the haptic feedback and/or the guide voice.

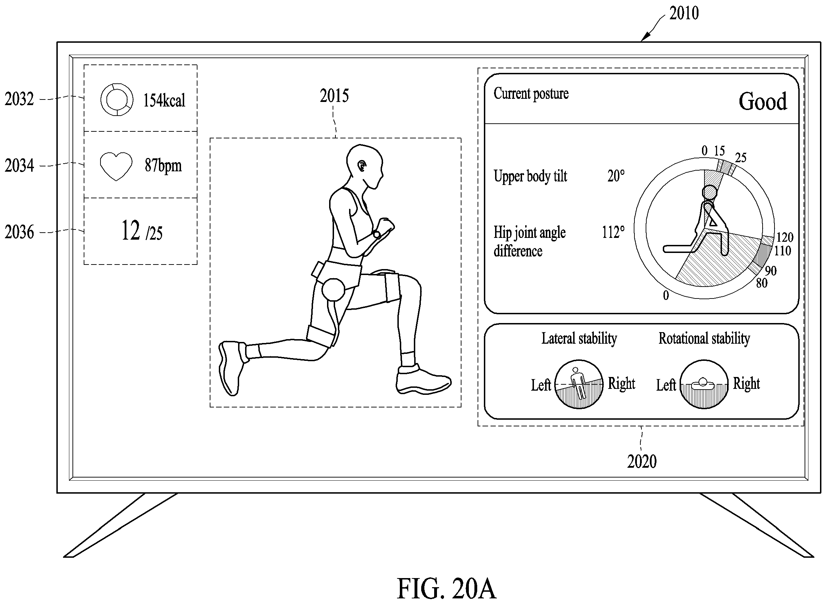

FIGS. 20A and 20B are diagrams illustrating examples of providing a GUI including visual guide objects through various electronic devices according to an example embodiment.

Referring to FIG. 20A, the electronic device 210 or the wearable device 100 may provide exercise posture evaluation information associated with exercise postures to a user in conjunction with a television (TV) (e.g., a smart TV). For example, on one screen 2010 provided through a display of the TV, there may be provided an exercise posture image obtained by capturing an image of an exercise posture of the user or a guide exercise posture image 2015, exercise posture evaluation information 2020 of the user including various visual guide objects, and other exercise-related information. For example, the exercise posture evaluation information 2020 including a first visual guide object that indicates an exercise posture reference for an exercise posture of the user and an exercise posture measurement value of the user and second visual guide objects that indicate physical movement stability may be provided. The other exercise-related information may include, for example, information about at least one of a calorie consumption estimate 2032 of calories consumed by an exercise up to a current point in time, the number 2034 of repetitions of an exercise posture performed up to the current point in time, or a heart rate 2036.

Referring to FIG. 20B, the electronic device 210 or the wearable device 100 may provide the user with exercise posture evaluation information associated with exercise postures in conjunction with a smartwatch 224. When the user performs an exercise posture with the wearable device 100 and the smartwatch 224 worn around the user, sensor data on a physical movement of the user may be obtained through the wearable device 100, and an evaluation may be performed on the exercise posture of the user based on the sensor data. A first visual guide object 2042 and a second visual guide object 2044 that indicate a result of the evaluation of the exercise posture may be provided to the user through a display of the smartwatch 224. In an example embodiment, the first visual guide object 2042 and the second visual guide object 2044 may be output on one screen of the display of the smartwatch 224 simultaneously or alternately at regular intervals.

It is to be understood that various embodiments of the disclosure and the terms used therein are not intended to limit the technological features set forth herein to particular embodiments and include various changes, equivalents, or replacements for a corresponding embodiment. In connection with the description of the drawings, like reference numerals may be used for similar or related components. It is to be understood that a singular form of a noun corresponding to an item may include one or more of the things unless the relevant context clearly indicates otherwise. As used herein, "A or B," "at least one of A and B," "at least one of A or B," "A, B, or C," "at least one of A, B, and C," and "at least one of A, B, or C," each of which may include any one of the items listed together in the corresponding one of the phrases, or all possible combinations thereof. Terms such as "first" or "second" may simply be used to distinguish the component from other components in question, and do not limit the components in other aspects (e.g., importance or order). It is to be understood that if an element (e.g., a first element) is referred to, with or without the term "operatively" or "communicatively," as "coupled with," "coupled to," "connected with," or "connected to" another element (e.g., a second element), it denotes that the element may be coupled with the other element directly (e.g., by wire), wirelessly, or via a third element.

As used in connection with certain example embodiments of the disclosure, the term "module" may include a unit implemented in hardware, software, or firmware, and may interchangeably be used with other terms, for example, "logic," "logic block," "part," or "circuitry." A module may be a single integral component, or a minimum unit or part thereof, adapted to perform one or more functions. For example, according to an example embodiment, the module may be implemented in the form of an application-specific integrated circuit (ASIC). Thus, each "module" herein may comprise circuitry.

Software may include a computer program, a piece of code, an instruction, or some combination thereof, to independently or collectively instruct or configure the processing device to operate as desired. Software and/or data may be embodied permanently or temporarily in any type of machine, component, physical or virtual equipment, computer storage medium or device, or in a propagated signal wave capable of providing instructions or data to or being interpreted by the processing device. The software also may be distributed over network-coupled computer systems so that the software is stored and executed in a distributed fashion. The software and data may be stored by one or more non-transitory computer-readable recording mediums (e.g., the memory 514). For example, a processor of a device or machine may invoke at least one of the one or more instructions stored in the storage medium and execute it. This allows the machine to be operated to perform at least one function according to the at least one instruction invoked. The one or more instructions may include code generated by a compiler or code executable by an interpreter. The machine-readable storage medium may be provided in the form of a non-transitory storage medium. Here, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to various embodiments, a method according to an example embodiment may be included and provided in a computer program product. The computer program product may be traded as a product between a seller and a buyer. The computer program product may be distributed in the form of a machine-readable storage medium (e.g., a compact disc read-only memory (CD-ROM)), or be distributed (e.g., downloaded or uploaded) online via an application store (e.g., PlayStore^{™}) or between two user devices (e.g., smartphones) directly. If distributed online, at least part of the computer program product may be temporarily generated or at least temporarily stored in the machine-readable storage medium, such as a memory of the manufacturer's server, a server of the application store, or a relay server.

According to various example embodiments, each component (e.g., a module or a program) of the components described above may include a single entity or multiple entities, and some of the multiple entities may be separately disposed in different components. According to various embodiments, one or more of the above-described components or operations may be omitted, or one or more other components or operations may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

While the disclosure has been illustrated and described with reference to various embodiments, it will be understood that the various embodiments are intended to be illustrative, not limiting. It will further be understood by those skilled in the art that various changes in form and detail may be made without departing from the true spirit and full scope of the disclosure, including the appended claims and their equivalents. It will also be understood that any of the embodiment(s) described herein may be used in conjunction with any other embodiment(s) described herein.

## Claims

1. An electronic device, comprising:
an input module, comprising input circuitry, configured to receive a user input for selection of an exercise program to be performed by a user of a wearable device;
a communication module, comprising communication circuitry, configured to receive, from the wearable device, sensor data comprising movement information of the user regarding an exercise to be performed by the user according to the selected exercise program;
at least one processor configured to determine an exercise posture measurement value of the user based on the sensor data, and to generate a first visual guide object comprising a first indicator indicating an exercise posture reference for the selected exercise program and at least one second indicator indicating the determined exercise posture measurement value; and
a display module, comprising a display, configured to output a graphical user interface(GUI) on which the first visual guide object is to be displayed.

2. The electronic device of claim 1, wherein the at least one second indicator comprises:
at least one of a second indicator indicating an exercise posture measurement value based on a previous exercise posture of the user and/or a second indicator indicating in real time an exercise posture measurement value based on a current exercise posture of the user.

3. The electronic device of claim 2, wherein the second indicator indicating the exercise posture measurement value based on the previous exercise posture comprises:
at least one of a maximum upper body tilt value of the user based on the previous exercise posture of the user or a maximum hip joint angle difference value between both hip joints of the user based on the previous exercise posture of the user.

4. The electronic device of claim 1, wherein the at least one second indicator has a color to be indicated in the first visual guide object that is to vary based on an evaluation reference range to which an exercise posture measurement value indicated by the at least one second indicator belongs.

5. The electronic device of claim 1, wherein the first indicator indicates an exercise posture reference range for the selected exercise program, and
the at least one second indicator indicates in real time an exercise posture measurement value of the user,
wherein, the at least one processor is configured so that when the exercise posture measurement value is in the exercise posture reference range indicated by the first indicator, the at least one second indicator is changed to a color corresponding to the exercise posture reference range.

6. The electronic device of claim 1, wherein the first indicator indicates a plurality of exercise posture reference ranges respectively corresponding to different exercise posture evaluation levels.

7. The electronic device of claim 1, wherein the first visual guide object further comprising:
at least one third indicator indicating an angle range indicated by a fixed reference line and an angle line of which an indication is changed based on an exercise posture measurement value of the user, and
wherein the at least one third indicator has a color to be indicated in the first visual guide obj ect that varies according to an evaluation reference range to which the exercise posture measurement value belongs.

8. The electronic device of claim 7, wherein the at least one third indicator comprises at least one of:
a third indicator indicating an angle range that is indicated by a reference line for an upper body tilt value of the user and an angle line corresponding to a maximum upper body tilt value of the user based on a previous exercise posture of the user; or
a third indicator indicating an angle range that is indicated by a reference line for a hip joint angle difference value between both hip joints of the user and an angle line corresponding to a maximum hip joint angle difference value between both hip joints of the user based on the previous exercise posture of the user.

9. The electronic device of claim 1, wherein the at least one processor is configured to:
determine an exercise posture measurement value corresponding to a physical movement stability of the user based on the sensor data, and generate at least one second visual guide object indicating the physical movement stability,
wherein the display module is configured to:
output a GUI on which the at least one second visual guide object is displayed.

10. The electronic device of claim 9, wherein the at least one second visual guide object comprises:
at least one of a second visual guide object indicating lateral stability of a physical movement of the user and/or a second visual guide object indicating rotational stability of the physical movement of the user.

11. The electronic device of claim 9, wherein the at least one second visual guide object comprises:
a fixed reference line, an avatar object that varies in tilt to be indicated based on an exercise posture measurement value corresponding to the physical movement stability, and a color object that varies in color based on an exercise posture evaluation level of the exercise posture measurement value corresponding to the physical movement stability.

12. The electronic device of claim 1, wherein the at least one processor is configured to:
control the communication module to transmit exercise posture evaluation information of the user to the wearable device, and allow the wearable device to provide the user with a feedback signal corresponding to the exercise posture evaluation information.

13. The electronic device of claim 1, wherein the first visual guide object further comprising:
an avatar object of which an operation to be displayed varies according to an exercise posture of the user, and
wherein, through the avatar object, exercise posture evaluation information determined for each exercise posture evaluation reference for the user is provided.

14. An operation method of an electronic device, comprising:
receiving a user input that selects an exercise program to be performed by a user wearing a wearable device;
receiving, from the wearable device, sensor data comprising movement information of the user during an exercise performed by the user according to the selected exercise program;
determining an exercise posture measurement value of the user based on the sensor data; and
outputting a graphical user interface (GUI) on which a first visual guide object comprising a first indicator indicating an exercise posture reference for the selected exercise program and at least one second indicator indicating the determined exercise posture measurement value is displayed.

15. A non-transitory computer-readable storage medium storing instructions that, when executed by a processor, cause the processor to perform the operation method of claim 14.
